(19) Europäisches Patentamt
European Patent Office
Office européen des brevets

(11) EP 2 945 609 B1

(12) **EUROPEAN PATENT SPECIFICATION**

(45) Date of publication and mention of the grant of the patent:
**27.02.2019 Bulletin 2019/09**

(21) Application number: **14701944.2**

(22) Date of filing: **20.01.2014**

(51) Int Cl.:
***A61K 8/36** (2006.01)* ***A61K 8/37** (2006.01)*
***A61K 8/49** (2006.01)* ***A61Q 19/00** (2006.01)*
***A61Q 19/02** (2006.01)* ***A61Q 17/04** (2006.01)*
***A61Q 19/04** (2006.01)* ***A61Q 19/08** (2006.01)*

(86) International application number:
**PCT/EP2014/051017**

(87) International publication number:
**WO 2014/111569 (24.07.2014 Gazette 2014/30)**

(54) **ANHYDROUS COSMETIC OR DERMATOLOGICAL COMPOSITION COMPRISING A MEROCYANINE AND AN OILY PHASE**

WASSERFREIE KOSMETISCHE ODER DERMATOLOGISCHE ZUSAMMENSETZUNG MIT EINEM MEROCYANIN UND EINER ÖLPHASE

COMPOSITION COSMÉTIQUE OU DERMATOLOGIQUE ANHYDRE COMPRENANT UNE MÉROCYANINE ET UNE PHASE HUILEUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.01.2013 FR 1350491**

(43) Date of publication of application:
**25.11.2015 Bulletin 2015/48**

(73) Proprietor: **L'Oréal**
**75008 Paris (FR)**

(72) Inventors:
- **ROUDOT, Angelina**
**F-94270 Le Kremlin Bicêtre (FR)**
- **CANDAU, Didier**
**F-91570 Bievres (FR)**

(74) Representative: **Le Blainvaux Bellegarde, Françoise**
**L'Oréal**
**Service DIPI**
**9 Rue Pierre Dreyfus**
**92110 Clichy (FR)**

(56) References cited:
**EP-A1- 2 236 173**
**WO-A1-2011/113718**
**WO-A1-2013/010590**
**WO-A2-2013/011094**
**FR-A1- 2 953 715**
**FR-A1- 2 957 249**
**FR-A1- 2 957 251**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 945 609 B1

Printed by Jouve, 75001 PARIS (FR)

## Description

The present invention relates to an anhydrous cosmetic or dermatological composition comprising, in a physiologically acceptable support:

a) at least one merocyanine compound of formula (3) defined herein below and
b) at least one oily phase comprising at least one oil chosen from hydrocarbon-based oils, silicone oils and fluoro oils, and mixtures thereof.

Another subject of the present invention consists of a non-therapeutic cosmetic process for caring for and/or making up a keratin material, comprising the application, to the surface of the said keratin material, of at least one composition according to the invention as defined above.

The invention also relates to a non-therapeutic cosmetic process for limiting the darkening of the skin and/or improving the colour and/or uniformity of the complexion, comprising the application, to the surface of the keratin material, of at least one composition as defined previously.

The invention also relates to a non-therapeutic cosmetic process for preventing and/or treating the signs of ageing of a keratin material, comprising the application, to the surface of the keratin material, of at least one composition as defined previously.

It is known that radiation with wavelengths of between 280 nm and 400 nm permits tanning of the human epidermis and that radiation with wavelengths of between 280 and 320 nm, known as UV-B rays, harms the development of a natural tan. Exposure is also liable to bring about a detrimental change in the biomechanical properties of the epidermis, which is reflected by the appearance of wrinkles, leading to premature ageing of the skin.

It is also known that UV-A rays with wavelengths of between 320 and 400 nm penetrate more deeply into the skin than UV-B rays. UV-A rays cause immediate and persistent browning of the skin. Daily exposure to UVA rays, even of short duration, under normal conditions can result in damage to the collagen fibres and the elastin, which is reflected by a modification in the microrelief of the skin, the appearance of wrinkles and uneven pigmentation (liver spots, lack of uniformity of the complexion).

Protection against UVA and UVB rays is thus necessary. An efficient photoprotective product should protect against both UVA and UVB radiation.

Many photoprotective compositions have been proposed to date to overcome the effects induced by UVA and/or UVB radiation. They generally contain organic or mineral UV-screening agents, which function according to their own chemical nature and according to their own properties by absorption, reflection or scattering of the UV radiation. They generally comprise mixtures of liposoluble organic screening agents and/or water-soluble UV screening agents in combination with metal oxide pigments, such as titanium dioxide or zinc oxide.

Many cosmetic compositions for limiting the darkening of the skin and improving the colour and uniformity of the complexion have been proposed to date. It is well known in the field of antisun products that such compositions may be obtained by using UV-screening agents, and in particular UVB-screening agents. Certain compositions may also contain UVA-screening agents. This screening system should cover UVB protection for the purpose of limiting and controlling the neosynthesis of melanin, which promotes the overall pigmentation, but should also cover UVA protection so as to limit and control the oxidation of the already-existing melanin leading to darkening of the skin colour.

However, it is extremely difficult to find a composition which contains a particular combination of UV-screening agents that would be especially suited to photoprotecting the skin and particularly to improving the quality of the skin as regards both the colour and its mechanical elasticity properties.

Advantageously, this improvement is particularly sought on already-pigmented skin so as not to increase the melanin pigmentary load or the structure of the melanin already present in the skin.

In point of fact, the majority of the organic UV-screening agents consist of aromatic compounds which absorb in the wavelength range between 280 and 370 nm. In addition to their power for screening out sunlight, the desired photoprotective compounds should also have good cosmetic properties, good solubility in the usual solvents and in particular in fatty substances such as oils, and also good chemical stability and good photostability, alone or in combination with other UV-screening agents. They should also be colourless or at least have a colour that is cosmetically acceptable to the consumer.

One of the main drawbacks known to date of these compositions is that these screening systems are insufficiently effective against UV rays and in particular against long UVA rays with wavelengths beyond 370 nm, for the purpose of controlling photo-induced pigmentation and its evolution by means of a system for screening out UV over the entire UV spectrum.

Among all the compounds that have been recommended for this purpose an advantageous family of UV-screening agents has been proposed, which consists of carbon-bearing merocyanine derivatives, which is described in patent US 4 195 999, patent application WO 2004/006 878, patent applications WO2008/090066, WO2011/113718,

WO2009/027258, WO2013/010590, WO2013/011094, WO2013/011480 and the documents IP COM JOURNAL N°000179675D published on February 23, 2009, IP COM JOURNAL N°000182396D published on April 29, IP COM JOURNAL N° 000189542D published on November 12, 2009, IP COM Journal N°IPCOM000011179D published on 03/04/2004. Some of these compounds may show the following drawbacks :

- relatively unsatisfactory solubility in the usual solvents and in particular in fatty substances such as oils which may require a laborious formulation process and/or may result in cosmetic drawbacks such as a greasy effect on application ;
- an unsatisfactory chemical stability and/or unsatisfactory photostability
- produce a color liable to discourage the consumer from using a cosmetic or dermatological composition containing them.

There is thus still a need to find new formulations comprising an oily phase and at least one merocyanine compound without the drawbacks as previously defined.. The Applicant has discovered, surprisingly, anhydrous compositions comprising at least one oily phase and at least one merocyanine compound of formula (3) defined hereinbelow, which make it possible to achieve this objective. Furthermore, the merocyanine compounds of formula (3) herein below, present surprinsingly the advantage to be significantly less colored than the merocyanine compounds as disclosed in the applications WO2008/090066 and WO2009/027258 as the compound MC11 (MC03).
Those discoveries form the basis of the present invention.
Thus, in accordance with one of the objects of the present invention, a cosmetic or dermatological composition is now proposed, comprising, in a physiologically acceptable support:

a) at least one merocyanine compound of formula (3) defined herein below and
b) at least one oily phase comprising at least one oil chosen from hydrocarbon-based oils, silicone oils and fluoro oils, and mixtures thereof.

Another subject of the present invention consists of a non-therapeutic cosmetic process for caring for and/or making up a keratin material, comprising the application, to the surface of the said keratin material, of at least one composition according to the invention as defined above.
The invention also relates to a non-therapeutic cosmetic process for limiting the darkening of the skin and/or improving the colour and/or uniformity of the complexion, comprising the application, to the surface of the keratin material, of at least one composition as defined previously.
The invention also relates to a non-therapeutic cosmetic process for preventing and/or treating the signs of ageing of a keratin material, comprising the application, to the surface of the keratin material, of at least one composition as defined previously.
Other characteristics, aspects and advantages of the invention will emerge on reading the detailed description that follows.
The expression "human keratin materials" means the skin (body, face, area around the eyes), hair, eyelashes, eyebrows, body hair, nails, lips or mucous membranes.
The term "physiologically acceptable" means compatible with the skin and/or its integuments, having a pleasant colour, odour and feel and not causing any unacceptable discomfort (stinging, tautness or redness) liable to discourage the consumer from using this composition.
The term "anhydrous composition" means a composition containing less than 1% by weight of water, or even less than 0.5% water, and especially free of water, the water not being added during the preparation of the composition but corresponding to the residual water provided by the mixed ingredients.
The term "between X and Y" means the range of values also including the limits X and Y.
According to the invention, the term "preventing" or "prevention" means reducing the risk of occurrence or slowing down the occurrence of a given phenomenon, namely, according to the present invention, the signs of ageing of a keratin material.

## MEROCYANINES

According to the present invention, the merocyanine compounds in accordance with the invention correspond to formula (3) below, and also the E/E- or E/Z-geometrical isomer forms thereof:

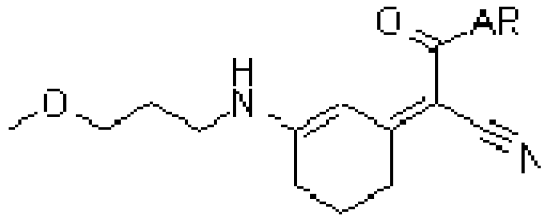

(3)

in which:

A is -O- or -NH;
R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, the said groups possibly being interrupted with one or more O.

The merocyanine compounds of the invention may be in their E/E- or E/Z-geometrical isomer forms.

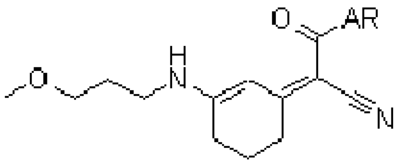

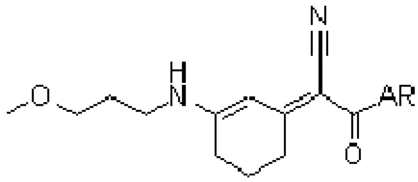

The even more preferential compounds of formula (3) are those in which:
A is -O-; R is a $C_1$-$C_{22}$ alkyl, which may be interrupted with one or more O.

Among the compounds of formula (3), use will be made more particularly of those chosen from the following group, and also the E/E- or E/Z- geometrical isomer forms thereof:

| | |
|---|---|
| 14 | 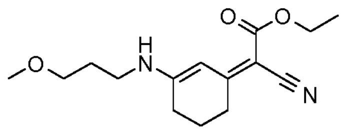 ethyl (2Z)-cyano{3-[(3-methoxypropyl)amino]cyclohex-2-en-1-ylidene}ethanoate |
| 15 | 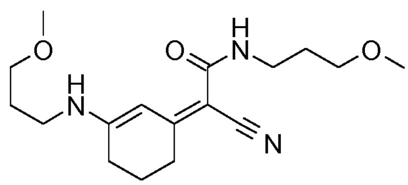 (2Z)-2-cyano-N-(3-methoxypropyl)-2-{3-[(3-methoxypropyl)amino]cyclohex-2-en-1-ylidene}ethanamide |
| 25 | 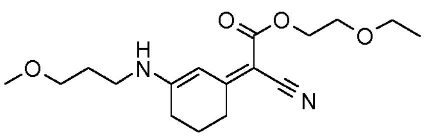 2-ethoxyethyl (2Z)-cyano{3-[(3-methoxypropyl)amino]cyclohex-2-en-1-ylidene}ethanoate |
| 27 | 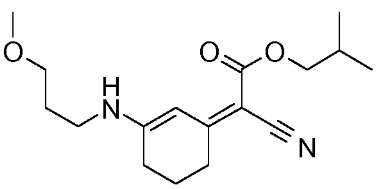 2-methylpropyl (2Z)-cyano{3-[(3-methoxypropyl)amino]cyclohex-2-en-1-ylidene}ethanoate |

(continued)

| | |
|---|---|
| 29 | 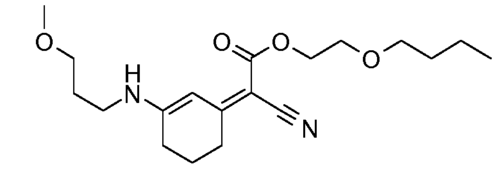<br>2-butoxyethyl (2Z)-cyano{3-[(3-methoxypropyl)amino]cyclohex-2-en-1-ylidene}ethanoate |
| 31 | 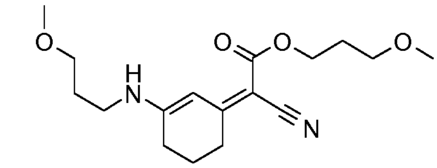<br>3-methoxypropyl (2Z)-cyano{3-[(3-methoxypropyl)amino]cyclohex-2-en-1-ylidene}ethanoate |
| 37 | 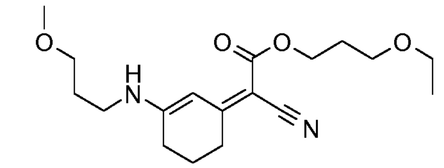<br>3-ethoxypropyl (2Z)-cyano{3-[(3-methoxypropyl)amino]cyclohex-2-en-1-ylidene}ethanoate |

According to a more particularly preferred mode of the invention, use will be made of the compound 2-ethoxyethyl (2Z)-cyano{3-[(3-methoxypropyl)amino]cyclohex-2-en-1-ylidene}ethanoate (25) in its E/E and/or E/Z geometrical configuration.

The E/Z form has the following structure:

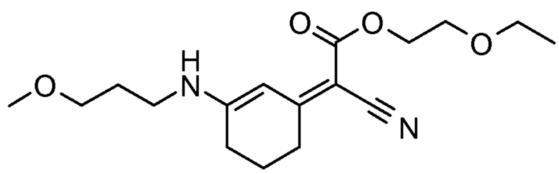

The E/E form has the following structure:

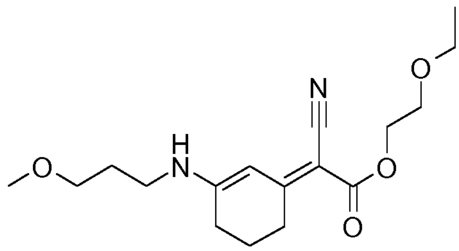

The screening merocyanines in accordance with the invention may be present in the compositions according to the invention in a concentration ranging from 0.1% to 10% by weight and preferentially from 0.2% to 5% by weight relative to the total weight of the composition.

The compounds of formula (3) may be prepared according to known processes, as described, for example, in J. Org. Chem. USSR (English translation) 26(8), p. 1562f (1990); J. Heterocycl. Chem. 33(3), p. 763-766 (1996); Khimiya Geterotsiklicheskikh Soedinenii 11, p. 1537-1543 (1984); Khimiya Geterotsiklicheskikh Soedinenii 3, p. 397-404 (1982); Chem.Heterocycl.Comp. (English translation) 24(8), 914-919 (1988) and in Synthetic Communications Vol. 33, No. 3, 2003, p 367-371.

The synthesis of the compounds used in the present invention is also described in US 2003/0 181 483 A1, WO 02/34710, Eur. J. Org. Chem. 2003, 2250-2253, J. Med. Chem. 1996, 39, 1112-1124 and J. Org. Chem., Vol. 37, No.

8, 1972, 1141-1145 as follows:

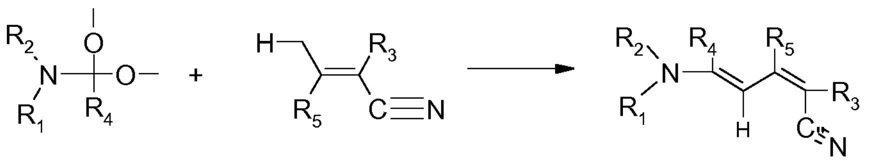

CH-acid vinylogen compounds are reacted with amide acetals.

In document J. Heterocyclic Chem., 27, 1990, 1143-1151, aminoacrylic acid esters or aminoacrylonitriles are reacted with ethoxymethylenecyanoacetates in ethanol to form the corresponding compounds of the present invention.

The compounds of formula (1) or (2) in which $R_4$ and $R_5$, on the one hand, or Rg and $R_{10}$, on the other hand, together form a carbocyclic ring containing 6 carbon atoms, respectively, may be prepared according to the protocols described in Pat. Appl. WO 2007/071 582, in IP.com Journal (2009), 9(5A), 29-30 IPCOM000182396D under the title "Process for producing 3-amino-2-cyclohexan-1-ylidene compounds" and in US-A-4 749 643 on column 13, line 66 - column 14, line 57 and the references cited in this regard.

## OILY PHASE

The compositions in accordance with the invention comprise at least one oily phase.

For the purposes of the invention, the term "oily phase" means a phase comprising at least one cosmetically acceptable water-immiscible oil other than aromatic hydrocarbons (for example toluene), and also all of the liposoluble and lipophilic ingredients and the cosmetically acceptable fatty substances used for the formulation of the compositions of the invention.

The term "oil" means any fatty substance which is in liquid form at room temperature (20 - 25°C) and at atmospheric pressure (760 mmHg).

An oil that is suitable for use in the invention may be volatile or non-volatile.

An oil that is suitable for use in the invention may be chosen from hydrocarbon-based oils, silicone oils and fluoro oils, and mixtures thereof.

A hydrocarbon-based oil that is suitable for use in the invention may be an animal hydrocarbon-based oil, a plant hydrocarbon-based oil, a mineral hydrocarbon-based oil or a synthetic hydrocarbon-based oil.

An oil that is suitable for use in the invention may be advantageously chosen from mineral hydrocarbon-based oils, plant hydrocarbon-based oils, synthetic hydrocarbon-based oils and silicone oils, and mixtures thereof.

For the purposes of the present invention, the term "silicone oil" means an oil comprising at least one silicon atom, and especially at least one Si-O group.

The term "hydrocarbon-based oil" means an oil comprising mainly hydrogen and carbon atoms.

The term "fluoro oil" means an oil comprising at least one fluorine atom.

A hydrocarbon-based oil that is suitable for use in the invention may also optionally comprise oxygen, nitrogen, sulfur and/or phosphorus atoms, for example in the form of hydroxyl, amine, amide, ester, ether or acid groups, and in particular in the form of hydroxyl, ester, ether or acid groups.

The oily phase generally comprises, in addition to the lipophilic UV-screening agent(s), at least one volatile or non-volatile hydrocarbon-based oil and/or one volatile and/or non-volatile silicone oil.

For the purposes of the invention, the term "volatile oil" means an oil that is capable of evaporating on contact with the skin or the keratin fibre in less than one hour, at room temperature and atmospheric pressure. The volatile oil(s) of the invention are volatile cosmetic oils which are liquid at room temperature and which have a non-zero vapour pressure, at room temperature and atmospheric pressure, ranging in particular from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" means an oil which remains on the skin or the keratin fibre, at room temperature and atmospheric pressure, for at least several hours and which in particular has a vapour pressure of less than $10^{-3}$ mmHg (0.13 Pa).

### Hydrocarbon-based oils

As non-volatile hydrocarbon-based oils that may be used according to the invention, mention may be made especially of:

(i) hydrocarbon-based oils of plant origin, such as glyceride triesters, which are generally triesters of fatty acids and of glycerol, the fatty acids of which can have varied chain lengths from $C_4$ to $C_{24}$, it being possible for these chains to be saturated or unsaturated and linear or branched; these oils are in particular wheat germ oil, sunflower oil, grape seed oil, sesame oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin seed oil, marrow oil, blackcurrant oil, evening primrose oil, millet oil, barley oil, quinoa oil, rye oil, safflower oil, candlenut oil, passionflower oil and musk rose oil; or also caprylic/capric acid triglycerides, such as those sold by Stéarineries Dubois or those sold under the names Miglyol 810®, 812® and 818® by Dynamit Nobel;

(ii) synthetic ethers containing from 10 to 40 carbon atoms;

(iii) linear or branched hydrocarbons of mineral or synthetic origin, such as petroleum jelly, polydecenes, hydrogenated polyisobutene such as Parleam, and squalane, and mixtures thereof;

(iv) synthetic esters, for instance oils of formula RCOOR' in which R represents a linear or branched fatty acid residue containing from 1 to 40 carbon atoms and R' represents a hydrocarbon-based chain that is especially branched, containing from 1 to 40 carbon atoms on condition that
R + R' is ≥10, for instance Purcellin oil (cetearyl octanoate), isopropyl myristate, isopropyl palmitate, $C_{12}$-$C_{15}$ alkyl benzoate, such as the product sold under the trade name Finsolv TN® or Witconol TN® by Witco or Tegosoft TN® by Evonik Goldschmidt, 2-ethylphenyl benzoate, such as the commercial product sold under the name X-Tend 226® by ISP, isopropyl lanolate, hexyl laurate, diisopropyl adipate, isononyl isononanoate, oleyl erucate, 2-ethylhexyl palmitate, isostearyl isostearate, diisopropyl sebacate, such as the product sold under the name of "Dub Dis" by Stearinerie Dubois, octanoates, decanoates or ricinoleates of alcohols or polyalcohols, such as propylene glycol dioctanoate; hydroxylated esters, such as isostearyl lactate or diisostearyl malate; and pentaerythritol esters; citrates or tartrates, such as di(linear $C_{12}$-$C_{13}$ alkyl) tartrates, such as those sold under the name Cosmacol ETI® by Enichem Augusta Industriale, and also di(linear $C_{14}$-$C_{15}$ alkyl) tartrates, such as those sold under the name Cosmacol ETL® by the same company; or acetates;

(v) fatty alcohols that are liquid at room temperature, containing a branched and/or unsaturated carbon-based chain containing from 12 to 26 carbon atoms, for instance octyldodecanol, isostearyl alcohol, oleyl alcohol, 2-hexyldecanol, 2-butyloctanol or 2-undecylpentadecanol;

(vi) higher fatty acids, such as oleic acid, linoleic acid or linolenic acid;

(vii) carbonates, such as dicaprylyl carbonate, such as the product sold under the name Cetiol CC® by Cognis;

(viii) fatty amides, such as isopropyl N-lauroyl sarcosinate, such as the product sold under the trade name Eldew SL 205® from Ajinomoto; and mixtures thereof.

Preference will more particularly be given, among the non-volatile hydrocarbon-based oils that may be used according to the invention, to glyceride triesters and in particular to caprylic/capric acid triglycerides, synthetic esters and in particular isononyl isononanoate, oleyl erucate, $C_{12}$-$C_{15}$ alkyl benzoate, 2-ethylphenyl benzoate and fatty alcohols, in particular octyldodecanol.

Mention may in particular be made, as volatile hydrocarbon-based oils that may be used according to the invention, of hydrocarbon-based oils containing from 8 to 16 carbon atoms and in particular of branched $C_8$-$C_{16}$ alkanes, such as $C_8$-$C_{16}$ isoalkanes of petroleum origin (also known as isoparaffins), such as isododecane (also known as 2,2,4,4,6-pentamethylheptane), isodecane or isohexadecane, the oils sold under the Isopar or Permethyl trade names, branched $C_8$-$C_{16}$ esters, isohexyl neopentanoate, and mixtures thereof.

Mention may also be made of the alkanes described in Cognis patent applications WO 2007/068 371 or WO 2008/155 059 (mixtures of distinct alkanes differing by at least one carbon). These alkanes are obtained from fatty alcohols, which are themselves obtained from coconut or palm oil. Mention may be made of the mixtures of n-undecane ($C_{11}$) and n-tridecane ($C_{13}$) obtained in Examples 1 and 2 of patent application WO 2008/155 059 from the company Cognis. Mention may also be made of n-dodecane ($C_{12}$) and n-tetradecane ($C_{14}$) sold by Sasol under the respective references Parafol 12-97 and Parafol 14-97®, and also mixtures thereof.

Use may also be made of other volatile hydrocarbon-based oils, such as petroleum distillates, in particular those sold under the name Shell Solt® by Shell. According to one embodiment, the volatile solvent is chosen from volatile hydrocarbon-based oils containing from 8 to 16 carbon atoms, and mixtures thereof.

b) Silicone oils

The non-volatile silicone oils may be chosen in particular from non-volatile polydimethylsiloxanes (PDMSs), polydimethylsiloxanes comprising alkyl or alkoxy groups which are pendent and/or at the end of the silicone chain, which groups each contain from 2 to 24 carbon atoms, or phenyl silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

Examples of volatile silicone oils that may be mentioned include volatile linear or cyclic silicone oils, especially those with a

viscosity ≤ 8 centistokes ($8 \times 10^{-6}$ $m^2/s$) and especially containing from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Mention may also be made of the volatile linear alkyltrisiloxane oils of general formula (I):

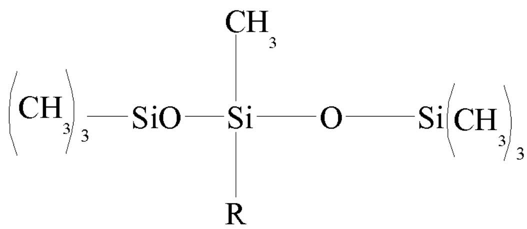

where R represents an alkyl group comprising from 2 to 4 carbon atoms, one or more hydrogen atoms of which may be substituted with a fluorine or chlorine atom.

Among the oils of general formula (I), mention may be made of: 3-butyl-1,1,1,3,5,5,5-heptamethyltrisiloxane, 3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

Fluoro oils

Use may also be made of volatile fluoro oils, such as nonafluoromethoxybutane, decafluoropentane, tetradecafluorohexane, dodecafluoropentane, and mixtures thereof.

An oily phase according to the invention may also comprise other fatty substances, mixed with or dissolved in the oil.

Another fatty substance that may be present in the oily phase may be, for example:

- a fatty acid chosen from fatty acids comprising from 8 to 30 carbon atoms, such as stearic acid, lauric acid, palmitic acid and oleic acid;
- a wax chosen from waxes such as lanolin, beeswax, carnauba or candelilla wax, paraffin waxes, lignite waxes, microcrystalline waxes, ceresin or ozokerite, or synthetic waxes, such as polyethylene waxes or Fischer-Tropsch waxes;
- a gum chosen from silicone gums (dimethiconol);
- a pasty compound, such as polymeric or non-polymeric silicone compounds, esters of a glycerol oligomer, arachidyl propionate, fatty acid triglycerides and derivatives thereof;
- and mixtures thereof.

According to a particular form of the invention, the overall oily phase, including all the lipophilic substances of the composition that are capable of being dissolved in this same phase, represents from 2% to 100% by weight and preferably from 10% to 90% by weight, relative to the total weight of the composition.

## ADDITIVES

### a) Additional water-insoluble screening agents

Preferentially, the compositions according to the invention contain, in addition to the merocyanine compound(s) of formula (3), one or more water-insoluble UV-screening agents.

The term "water-insoluble UV-screening agent" means any organic or mineral compound that is capable of screening out UV radiation in the wavelength range from 280 to 400 nm by absorption, reflection and/or scattering; the said compound having a solubility in water at 25°C of less than 1% by weight or even less than 0.5% by weight or further still less than 0.1% by weight.

The water-insoluble UV-screening agents are generally chosen from organic UV-screening agents that are lipophilic or insoluble in oils and/or mineral screening agents.

The term "lipophilic screening agent" means any cosmetic or dermatological organic or mineral compound for screening out UV radiation, which can be fully dissolved in molecular state in a liquid fatty phase or which can be dissolved in colloidal form (for example in micellar form) in a liquid fatty phase.

The term "oil-insoluble UV-screening agent" means any cosmetic or dermatological organic or mineral compound for screening out UV radiation which has a solubility of less than 0.5% by weight in the majority of organic solvents such as liquid paraffin, fatty alkyl benzoates and fatty acid triglycerides, for example Miglyol 812® sold by the company Dynamit Nobel. This solubility, determined at 70°C, is defined as the amount of product in solution in the solvent at equilibrium with an excess of solid in suspension after returning to room temperature. It may be readily evaluated in the laboratory.

The additional organic UV-screening agents are chosen in particular from cinnamic compounds; anthranilates; salicylic compounds; dibenzoylmethane compounds; benzylidenecamphor compounds; benzophenone compounds; β,β-diphenylacrylate compounds; triazine compounds; benzotriazole compounds; benzalmalonate compounds, in particular those cited in patent US 5 624 663; benzimidazole compounds; imidazolines; p-aminobenzoic acid (PABA) compounds; methylenebis(hydroxyphenylbenzotriazole) compounds, such as described in patent applications US 5 237 071, US 5 166 355, GB 2 303 549, DE 197 26 184 and EP 893 119; benzoxazole compounds, as described in patent applications EP 0 832 642, EP 1 027 883, EP 1 300 137 and DE 101 62 844; screening polymers and screening silicones, such as those described in particular in patent application WO 93/04665; α-alkylstyrene-based dimers, such as those described in patent application DE 198 55 649; 4,4-diarylbutadiene compounds, as described in patent applications EP 0 967 200, DE 197 46 654, DE 197 55 649, EP-A-1 008 586, EP 1 133 980 and EP 133 981, and mixtures thereof.

As examples of organic photoprotective agents, mention may be made of those denoted hereinbelow under their INCI name:

Cinnamic compounds:

Ethylhexyl Methoxycinnamate, sold in particular under the trade name Parsol MCX® by DSM Nutritional Products,
Isopropyl Methoxycinnamate,
Isoamyl p-methoxycinnamate sold under the trade name Neo Heliopan E 1000® by Symrise,
DEA Methoxycinnamate,
Diisopropyl Methyl Cinnamate,
Glyceryl Ethylhexanoate Dimethoxycinnamate.

Dibenzoylmethane compounds:

Butylmethoxydibenzoylmethane sold in particular under the trade name Parsol 1789® by DSM Nutritional Products,
Isopropyldibenzoylmethane.

para-Aminobenzoic compounds:

PABA,
Ethyl PABA,
Ethyl Dihydroxypropyl PABA,
Ethylhexyl Dimethyl PABA, sold in particular under the name Escalol 507® by ISP,
Glyceryl PABA,
PEG-25 PABA, sold under the name Uvinul P 25® by BASF.

Salicylic compounds:

Homosalate, sold under the name Eusolex HMS® by Rona/EM Industries,
Ethylhexyl Salicylate, sold under the name Neo Heliopan OS® by Symrise,
Dipropylene Glycol Salicylate, sold under the name Dipsal® by Scher,
TEA Salicylate, sold under the name Neo Heliopan TS® by Symrise.

β,β-Diphenylacrylate compounds:

Octocrylene, sold in particular under the trade name Uvinul N 539® by BASF, Etocrylene, sold in particular under the trade name Uvinul N 35® by BASF.

Benzophenone compounds:

Benzophenone-1, sold under the trade name Uvinul 400® by BASF,
Benzophenone-2, sold under the trade name Uvinul D 50® by BASF,
Benzophenone-3 or Oxybenzone, sold under the trade name Uvinul M 40® by BASF,
Benzophenone-4, sold under the trade name Uvinul MS 40® by BASF,
Benzophenone-5,
Benzophenone-6, sold under the trade name Helisorb 11® by Norquay,
Benzophenone-8, sold under the trade name Spectra-Sorb UV-24® by American Cyanamid,
Benzophenone-9, sold under the trade name Uvinul DS 49® by BASF, Benzophenone-12,
n-Hexyl 2-(4-diethylamino-2-hydroxybenzoyl)benzoate, sold under the trade name Uvinul A Plus® or, as a mixture with octyl methoxycinnamate, under the trade name Uvinul A Plus B® by BASF,
1,1'-(1,4-Piperazinediyl)bis[1-[2-[4-(diethylamino)-2-hydroxybenzoyl]phenyl]methanone] (CAS 919803-06-8), such as described in patent application WO 2007/071 584; this compound advantageously being used in micronized form (mean size of 0.02 to 2 μm), which may be obtained, for example, according to the micronization process described in patent applications GB-A-2 303 549 and EP-A-893 119, and in particular in the form of an aqueous dispersion.

Benzylidenecamphor compounds:

3-Benzylidene Camphor, manufactured under the name Mexoryl SD® by Chimex, 4-Methylbenzylidene Camphor, sold under the name Eusolex 6300® by Merck, Benzylidene Camphor Sulfonic Acid, manufactured under the name Mexoryl SL® by Chimex,
Camphor Benzalkonium Methosulfate, manufactured under the name Mexoryl SO® by Chimex,
Terephthalylidene Dicamphor Sulfonic Acid, manufactured under the name Mexoryl SX® by Chimex,
Polyacrylamidomethyl Benzylidene Camphor, manufactured under the name Mexoryl SW® by Chimex.

Phenylbenzimidazole compounds:

Phenylbenzimidazole Sulfonic Acid, sold in particular under the trade name Eusolex 232® by Merck.

Bis-benzazolyl compounds:

Disodium Phenyl Dibenzimidazole Tetrasulfonate, sold under the trade name Neo Heliopan AP® by Haarmann and Reimer.

Phenylbenzotriazole compounds:

Drometrizole Trisiloxane, sold under the name Silatrizole® by Rhodia Chimie.

Methylenebis(hydroxyphenylbenzotriazole) compounds:

Methylenebis(benzotriazolyl)tetramethylbutylphenol especially in solid form, for instance the product sold under the trade name Mixxim BB/100® by Fairmount Chemical or in the form of an aqueous dispersion of micronized particles with a mean particle size ranging from 0.01 to 5 μm, more preferentially from 0.01 to 2 μm and more particularly from 0.020 to 2 μm with at least one alkylpolyglycoside surfactant of structure $C_nH_{2n+1}O(C_6H_{10}O_5)_xH$, in which n is an integer

from 8 to 16 and x is the mean degree of polymerization of the ($C_6H_{10}O_5$) unit and ranges from 1.4 to 1.6, such as described in patent GB-A-2 303 549, sold in particular under the trade name Tinosorb M® by BASF, or in the form of an aqueous dispersion of micronized particles with a mean particle size ranging from 0.02 to 2 μm, more preferably from 0.01 to 1.5 μm and more particularly from 0.02 to 1 μm, in the presence of at least one polyglyceryl mono($C_8$-$C_{20}$)alkyl ester with a degree of glycerol polymerization of at least 5, such as the aqueous dispersions described in patent application WO 2009/063 392.

Triazine compounds:

Bis-Ethylhexyloxyphenol Methoxyphenyl Triazine, sold under the trade name Tinosorb S® by BASF,
Ethylhexyl Triazone, sold in particular under the trade name Uvinul T150® by BASF,
Diethylhexyl Butamido Triazone, sold under the trade name Uvasorb HEB® by Sigma 3V,
2,4,6-tris(dineopentyl 4'-aminobenzalmalonate)-s-triazine,
2,4,6-tris(diisobutyl 4'-aminobenzalmalonate)-s-triazine;
2,4-bis(n-butyl 4'-aminobenzoate)-6-(aminopropyltrisiloxane)-s-triazine, 2,4-bis(dineopentyl 4'-aminobenzalmalonate)-6-(n-butyl 4'-aminobenzoate)-s-triazine,
symmetrical triazine screening agents substituted with naphthalenyl groups or polyphenyl groups described in patent US 6 225 467, patent application WO 2004/085 412 (see compounds 6 and 9) or the document "Symmetrical Triazine Derivatives", IP.COM IPCOM000031257 Journal, INC, West Henrietta, NY, US (20 September 2004), in particular 2,4,6-tris(diphenyl)triazine and 2,4,6-tris(terphenyl)triazine, which is also mentioned in patent applications WO 06/035 000, WO 06/034 982, WO 06/034 991, WO 06/035 007, WO 2006/034 992 and WO 2006/034 985, these compounds advantageously being used in micronized form (mean particle size of 0.02 to 3 μm), which may be obtained, for example, according to the micronization process described in patent applications GB-A-2 303 549 and EP-A-893 119, and in particular in the form of an aqueous dispersion; silicone triazines substituted with two aminobenzoate groups, as described in patent EP 0 841 341, in particular 2,4-bis(n-butyl 4'-aminobenzalmalonate)-6-[(3-{1,3,3,3-tetramethyl-1-[(trimethylsilyl)oxy]disiloxanyl}propyl)amino]-s-triazine.

Anthranilic compounds:

Menthyl Anthranilate, sold under the trade name Neo Heliopan MA® by Symrise.
Imidazoline compounds:
Ethylhexyl dimethoxybenzylidene dioxoimidazoline propionate.

Benzalmalonate compounds:

Polyorganosiloxane comprising benzalmalonate functional groups, such as Polysilicone-15, sold under the trade name Parsol SLX® by Hoffmann-LaRoche.

4,4-Diarylbutadiene compounds:

1,1-Dicarboxy(2,2'-dimethylpropyl)-4,4-diphenylbutadiene.

Benzoxazole compounds:

2,4-Bis[4-[5-(1,1-dimethylpropyl)benzoxazol-2-yl]phenylimino]-6-[(2-ethylhexyl)imino]-1,3,5-triazine, sold under the name of Uvasorb K2A® by Sigma 3V.
The preferred organic screening agents are chosen from:

Ethylhexyl Methoxycinnamate,
Ethylhexyl salicylate,
Homosalate,
Butyl Methoxy Dibenzoylmethane,
Octocrylene,
Phenylbenzimidazole Sulfonic Acid,
Benzophenone-3,
Benzophenone-4,
Benzophenone-5,
n-Hexyl 2-(4-diethylamino-2-hydroxybenzoyl)benzoate,

4-Methylbenzylidene Camphor,
Terephthalylidene Dicamphor Sulfonic Acid,
Disodium Phenyl Dibenzimidazole Tetrasulfonate,
Methylene Bis-Benzotriazolyl Tetramethylbutylphenol,
Bis-Ethylhexyloxyphenyl Methoxyphenyl Triazine,
Ethylhexyl Triazone,
Diethylhexyl Butamido Triazone,
2,4,6-Tris(dineopentyl 4'-aminobenzalmalonate)-s-triazine,
2,4,6-Tris(diisobutyl 4'-aminobenzalmalonate)-s-triazine,
2,4-bis(n-butyl 4'-aminobenzoate)-6-(aminopropyltrisiloxane)-s-triazine,
2,4-Bis(dineopentyl 4'-aminobenzalmalonate)-6-(n-butyl 4'-aminobenzoate)-s-triazine,
2,4,6-Tris(diphenyl)triazine,
2,4,6-Tris(terphenyl)triazine,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
Drometrizole trisiloxane,
Polysilicone-15, 1,1-Dicarboxy(2,2'-dimethylpropyl)-4,4-diphenylbutadiene,
2,4-Bis[4[5-(1,1-dimethylpropyl)benzoxazol-2-yl]phenylimino]-6-[(2-ethylhexyl)imino-1,3,5-triazine,
and mixtures thereof.

The particularly preferred organic screening agents are chosen from:

Ethylhexyl salicylate,
Homosalate,
Butylmethoxydibenzoylmethane,
Octocrylene,
n-Hexyl 2-(4-diethylamino-2-hydroxybenzoyl)benzoate,
Terephthalylidene Dicamphor Sulfonic Acid,
Bis(ethylhexyloxyphenol)methoxyphenyltriazine,
Ethylhexyl Triazone,
Diethylhexyl Butamido Triazone,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
Drometrizole Trisiloxane,
and mixtures thereof.

The additional mineral UV-screening agents used in accordance with the present invention are metal oxide pigments. More preferentially, the mineral UV-screening agents are metal oxide particles with a mean elementary particle size of less than or equal to 0.5 μm, more preferentially between 0.005 and 0.5 μm, even more preferentially between 0.01 and 0.2 μm, better still between 0.01 and 0.1 μm and more particularly between 0.015 and 0.05 μm.
They may be selected in particular from titanium oxide, zinc oxide, iron oxide, zirconium oxide and cerium oxide, or mixtures thereof.
Such coated or uncoated metal oxide pigments are described in particular in patent application EP-A-0 518 773. Commercial pigments that may be mentioned include the products sold by the companies Sachtleben Pigments, Tayca, Merck and Degussa.
The metal oxide pigments may be coated or uncoated and preferably coated with at least one hydrophobic material.
The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.
The coated pigments are more particularly titanium oxides that have been coated:

- with silica, such as the product Sunveil® from the company Ikeda,
- with silica and iron oxide, such as the product Sunveil F® from the company Ikeda,
- with silica and alumina, such as the products Microtitanium Dioxide MT 500 SA® and Microtitanium Dioxide MT 100 SA from the company Tayca and Tioveil from the company Tioxide,

- with alumina, such as the products Tipaque TTO-55 (B)® and Tipaque TTO-55 (A)® from the company Ishihara and UVT 14/4 from the company Sachtleben Pigments,
- with alumina and aluminium stearate, such as the products Microtitanium Dioxide MT 100 T®, MT 100 TX®, MT 100 Z® and MT-01® from the company Tayca, the products Solaveil CT-10 W® and Solaveil CT 100® from the company Uniqema and the product Eusolex T-AVO® from the company Merck,
- with silica, alumina and alginic acid, such as the product MT-100 AQ® from the company Tayca,
- with alumina and aluminium laurate, such as the product Microtitanium Dioxide MT 100 S® from the company Tayca,
- with iron oxide and iron stearate, such as the product Microtitanium Dioxide MT 100 F® from the company Tayca,
- with zinc oxide and zinc stearate, such as the product BR 351® from the company Tayca,
- with silica and alumina and treated with a silicone, such as the products Microtitanium Dioxide MT 600 SAS®, Microtitanium Dioxide MT 500 SAS® or Microtitanium Dioxide MT 100 SAS® from the company Tayca,
- with silica, alumina and aluminium stearate and treated with a silicone, such as the product STT-30-DS® from the company Titan Kogyo,
- with silica and treated with a silicone, such as the product UV-Titan X 195® from the company Sachtleben Pigments,
- with alumina and treated with a silicone, such as the products Tipaque TTO-55 (S)® from the company Ishihara or UV Titan M 262® from the company Sachtleben Pigments,
- with triethanolamine, such as the product STT-65-S from the company Titan Kogyo,
- with stearic acid, such as the product Tipaque TTO-55 (C)® from the company Ishihara,
- with sodium hexametaphosphate, such as the product Microtitanium Dioxide MT 150 W® from the company Tayca,
- $TiO_2$ treated with octyltrimethylsilane, sold under the trade name T 805® by the company Degussa Silices,
- $TiO_2$ treated with a polydimethylsiloxane, sold under the trade name 70250 Cardre UF TiO2SI3® by the company Cardre,
- anatase/rutile $TiO_2$ treated with a polydimethylhydrogenosiloxane, sold under the trade name Microtitanium Dioxide USP Grade Hydrophobic® by the company Color Techniques.

Mention may also be made of $TiO_2$ pigments doped with at least one transition metal such as iron, zinc or manganese and more particularly manganese. Preferably, the said doped pigments are in the form of an oily dispersion. The oil present in the oily dispersion is preferably chosen from triglycerides including those of capric/caprylic acids. The oily dispersion of titanium oxide particles may also comprise one or more dispersants, for instance a sorbitan ester, for instance sorbitan isostearate, or a polyoxyalkylenated fatty acid ester of glycerol, for instance TRI-PPG3 myristyl ether citrate and polyglyceryl-3 polyricinoleate. Preferably, the oily dispersion of titanium oxide particles comprises at least one dispersant chosen from polyoxyalkylenated fatty acid esters of glycerol. Mention may be made more particularly of the oily dispersion of $TiO_2$ particles doped with manganese in capric/caprylic acid triglycerides in the presence of TRI-PPG-3 myristyl ether citrate and polyglyceryl-3 polyricinoleate and sorbitan isostearate having the INCI name: titanium dioxide (and) TRI-PPG-3 myristyl ether citrate (and) polyglyceryl-3 ricinoleate (and) sorbitan isostearate, for instance the product sold under the trade name Optisol TD50® by the company Croda.

The uncoated titanium oxide pigments are sold, for example, by the company Tayca under the trade names Microtitanium Dioxide MT 500 B or Microtitanium Dioxide MT 600 B®, by the company Degussa under the name P 25, by the company Wackher under the name Transparent titanium oxide PW®, by the company Miyoshi Kasei under the name UFTR®, by the company Tomen under the name ITS® and by the company Tioxide under the name Tioveil AQ.

The uncoated zinc oxide pigments are for example:

- those sold under the name Z-Cote by the company Sunsmart;
- those sold under the name Nanox® by the company Elementis;
- those sold under the name Nanogard WCD 2025® by the company Nanophase Technologies.

The coated zinc oxide pigments are for example:

- those sold under the name Zinc Oxide CS-5® by the company Toshibi (ZnO coated with polymethylhydrogenosiloxane);
- those sold under the name Nanogard Zinc Oxide FN® by the company Nanophase Technologies (as a 40% dispersion in Finsolv TN®, $C_{12}$-$C_{15}$ alkyl benzoate);
- those sold under the name Daitopersion Zn-30® and Daitopersion Zn-50® by the company Daito (dispersions in cyclopolymethylsiloxane/oxyethylenated polydimethylsiloxane, containing 30% or 50% of zinc oxides coated with silica and polymethylhydrogenosiloxane);
- those sold under the name NFD Ultrafine ZnO® by the company Daikin (ZnO coated with perfluoroalkyl phosphate and copolymer based on perfluoroalkylethyl as a dispersion in cyclopentasiloxane);
- those sold under the name SPD-Z1® by the company Shin-Etsu (ZnO coated with silicone-grafted acrylic polymer,

dispersed in cyclodimethylsiloxane);

- those sold under the name Escalol Z100® by the company ISP (alumina-treated ZnO dispersed in an ethylhexyl methoxycinnamate/PVP-hexadecene copolymer/methicone mixture);
- those sold under the name Fuji ZnO-SMS-10® by the company Fuji Pigment (ZnO coated with silica and polymethylsilsesquioxane);
- those sold under the name Nanox Gel TN® by the company Elementis (ZnO dispersed at a concentration of 55% in $C_{12}$-$C_{15}$ alkyl benzoate with hydroxystearic acid polycondensate).

The uncoated cerium oxide pigments may be, for example, those sold under the name Colloidal Cerium Oxide® by the company Rhone-Poulenc.

The uncoated iron oxide pigments are sold, for example, by the company Arnaud under the names Nanogard WCD 2002® (FE 45B®), Nanogard Iron FE 45 BL AQ, Nanogard FE 45R AQ® and Nanogard WCD 2006® (FE 45R®) or by the company Mitsubishi under the name TY-220®.

The coated iron oxide pigments are sold, for example, by the company Arnaud under the names Nanogard WCD 2008 (FE 45B FN)®, Nanogard WCD 2009® (FE 45B 556®), Nanogard FE 45 BL 345® and Nanogard FE 45 BL® or by the company BASF under the name Transparent Iron Oxide®.

Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A®, and also the mixture of titanium dioxide and zinc dioxide coated with alumina, silica and silicone, such as the product M 261® sold by the company Sachtleben Pigments, or coated with alumina, silica and glycerol, such as the product M 211® sold by the company Sachtleben Pigments.

According to the invention, coated or uncoated titanium dioxide pigments are particularly preferred, and even more particularly titanium dioxide pigments coated with at least one hydrophobic material.

The additional UV-screening agents according to the invention are preferably present in the compositions according to the invention in proportions ranging from 0.1% to 50% by weight, relative to the total weight of the composition, and preferably ranging from 2% to 30% by weight, relative to the total weight of the composition.

**b) <u>Other additives</u>**

The compositions in accordance with the present invention may also comprise conventional cosmetic adjuvants chosen in particular from organic solvents, ionic or nonionic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants, or any other ingredient commonly used in the cosmetic and/or dermatological field.

Among the organic solvents that may be mentioned are lower alcohols and polyols. These polyols may be chosen from glycols and glycol ethers, for instance ethylene glycol, propylene glycol, butylene glycol, dipropylene glycol or diethylene glycol.

Thickeners that may be mentioned include the Pemulen products (acrylate/$C_{10}$-$C_{30}$-alkyl acrylate copolymer) (Pemulen TR1® or Pemulen TR2®); polysaccharides and especially gums such as xanthan gum, and mixtures thereof.

Among the active agents for caring for keratin materials such as the skin, the lips, the scalp, the hair, the eyelashes or the nails, examples that may be mentioned include:

- vitamins and derivatives or precursors thereof, alone or as mixtures;
- antioxidants;
- free-radical scavengers;
- antipollution agents;
- self-tanning agents;
- antiglycation agents;
- calmatives;
- deodorants;
- essential oils;
- NO-synthase inhibitors;
- agents for stimulating the synthesis of dermal or epidermal macromolecules and/or which prevent their decomposition;
- agents for stimulating the proliferation of fibroblasts;
- agents for stimulating the proliferation of keratinocytes;
- muscle relaxants;
- refreshing agents;

- tensioning agents;
- mattifying agents;
- depigmenting agents;
- propigmenting agents;
- keratolytic agents;
- desquamating agents;
- moisturizers;
- antiinflammatory agents;
- antimicrobial agents;
- slimming agents;
- agents acting on the energy metabolism of cells;
- insect repellents;
- substance P or CGRP antagonists;
- hair-loss counteractants;
- antiwrinkle agents;
- antiageing agents.

A person skilled in the art will select the said active principle(s) according to the effect desired on the skin, the hair, the eyelashes, the eyebrows or the nails.

Needless to say, a person skilled in the art will take care to select the abovementioned optional additional compound(s) and/or the amounts thereof such that the advantageous properties intrinsically associated with the compositions in accordance with the invention are not, or are not substantially, adversely affected by the envisaged addition(s).

## GALENICAL FORMS

The compositions according to the invention may be prepared according to the techniques that are well known to those skilled in the art for manufacturing anhydrous compositions. They may be in the form of a fluid lotion, a gel or a compact composition, for example a hot-cast composition. They may optionally be in the form of a loose or compacted powder.

Preferably, the compositions according to the invention are in the form of a fluid composition or a gel.

For the purposes of the invention, the term "fluid composition" means a composition that is not in solid form and whose viscosity, measured using a Rheomat 180 viscometer at 25°C at a spin speed of 200 rpm after 30 seconds of rotation, is less than 0.5 Pa.s, more preferentially less than 0.2 Pa.s and more particularly ranging from 0.0001 Pa.s to 0.1 Pa.s.

According to one particularly preferred form of the invention, the compositions will be transparent and will preferably have a turbidity of less than 1000 NTU (nephelometric turbidity units) at 25°C, preferably less than 50 NTU at 25°C and even more preferentially less than 15 NTU, measured using a 2100P turbidimeter machine from the company Hach.

According to a particular form of the invention, the composition of the invention is a fluid composition also comprising:

a) at least one linear $C_1$-$C_4$ monoalkanol and
b) at least one lipophilic polyamide polycondensate.

## LIPOPHILIC POLYAMIDE POLYCONDENSATE

For the purposes of the invention, the term "polycondensate" means a polymer obtained by polycondensation, i.e. by chemical reaction between monomers bearing different functional groups chosen in particular from acid, alcohol and amine functions.

For the purposes of the invention, the term "polymer" means a compound containing at least two repeating units, preferably at least three repeating units and better still ten repeating units.

The term "lipophilic polymer" means any polymer that can be fully dissolved in molecular form in a liquid fatty phase or that can be dissolved in colloidal form (for example in micellar form) in a liquid fatty phase.

The lipophilic polyamide polycondensate(s) are preferably present in the compositions of the invention in concentrations ranging from 0.1% to 15% by weight and more preferentially from 1% to 8% by weight relative to the total weight of the composition.

The lipophilic polyamide polycondensates may be chosen especially from polyamide polymers comprising a) a polymer backbone containing hydrocarbon-based repeating units bearing at least one non-pendent amide unit, and

optionally b) at least one pendent fatty chain and/or at least one terminal fatty chain, which are optionally functionalized, comprising at least four carbon atoms and being attached to these hydrocarbon-based units.

For the purposes of the invention, the term "functionalized chains" means an alkyl chain comprising one or more functional groups or reagents chosen especially from amide, hydroxyl, ether, oxyalkylene or polyoxyalkylene, halogen, including fluoro or perfluoro groups, and ester, siloxane and polysiloxane groups. In addition, the hydrogen atoms of one or more fatty chains may be at least partially replaced with fluorine atoms.

For the purposes of the invention, the term "hydrocarbon-based repeating units" means a unit comprising from 2 to 80 carbon atoms and preferably from 2 to 60 carbon atoms, bearing hydrogen atoms and optionally oxygen atoms, which may be linear, branched or cyclic, and saturated or unsaturated. These units each also comprise at least one amide group that is advantageously non-pendent, which is in the polymer backbone.

The pendent chains are advantageously bonded directly to at least one of the nitrogen atoms of the polymer backbone.

The lipophilic polyamide polycondensate may comprise between the hydrocarbon-based units silicone units or oxyalkylene units.

In addition, the lipophilic polyamide polycondensate of the composition of the invention advantageously comprises from 40% to 98% of fatty chains relative to the total number of amide units and fatty chains, and better still from 50% to 95%.

The pendent fatty chains are preferably bonded to at least one of the nitrogen atoms of the amide units of the polymer. In particular, the fatty chains of this polyamide represent from 40% to 98% of the total number of amide units and of fatty chains, and better still from 50% to 95%.

Advantageously, the lipophilic polyamide polycondensate has a weight-average molecular mass of less than 100 000 (especially ranging from 1000 to 100 000), in particular less than 50 000 (especially ranging from 1000 to 50 000) and more particularly ranging from 1000 to 30 000, preferably from 2000 to 20 000 and better still from 2000 to 10 000.

The lipophilic polyamide polycondensate is insoluble in water, especially at 25°C. In particular, it contains no ionic groups.

As preferred lipophilic polyamide polycondensates that may be used in the invention, mention may be made of polyamides branched with pendent fatty chains and/or terminal fatty chains containing from 6 to 120 carbon atoms and better still from 8 to 120 and in particular from 12 to 68 carbon atoms, each terminal fatty chain being bonded to the polyamide backbone via at least one bonding group L. The bonding group L may be chosen from ester, ether, amine, urea, urethane, thioester, thioether, thiourea and thiourethane groups. Preferably, these polymers comprise a fatty chain at each end of the polyamide backbone.

These polymers are preferably polymers resulting from a polycondensation between a dicarboxylic acid containing at least 32 carbon atoms (in particular containing from 32 to 44 carbon atoms) and an amine chosen from diamines containing at least 2 carbon atoms (in particular from 2 to 36 carbon atoms) and triamines containing at least 2 carbon atoms (in particular from 2 to 36 carbon atoms). The diacid is preferably a dimer of a fatty acid containing ethylenic unsaturation containing at least 16 carbon atoms, preferably from 16 to 24 carbon atoms, for instance oleic acid, linoleic acid or linolenic acid. The diamine is preferably ethylenediamine, hexylenediamine or hexamethylenediamine. The triamine is, for example, ethylenetriamine. For the polymers comprising one or two terminal carboxylic acid groups, it is advantageous to esterify them with a monoalcohol containing at least four carbon atoms, preferably from 10 to 36 carbon atoms, better still from 12 to 24 and even better from 16 to 24, for example 18 carbon atoms.

The lipophilic polyamide polycondensate of the composition according to the invention may be chosen in particular from the polymers of formula (A) below:

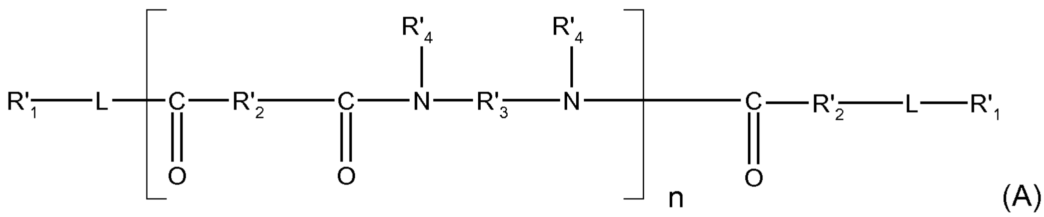

(A)

in which:

n is an integer ranging from 1 to 30;

R'$_1$ represents independently in each case a fatty chain and is chosen from an alkyl or alkenyl group containing at least 1 carbon atom and especially from 4 to 24 carbon atoms;

R'$_2$ represents independently in each case a hydrocarbon-based radical comprising from 1 to 52 carbon atoms;

R'$_3$ represents independently in each case an organic group comprising at least one atom chosen from carbon,

hydrogen and nitrogen atoms, on condition that R'$_3$ comprises at least three carbon atoms;
R'$_4$ represents independently in each case: a hydrogen atom, an alkyl group comprising from 1 to 10 carbon atoms, or a direct bond to at least one group chosen from R'$_3$ and another R'$_4$ such that when the said group is another R'$_4$, the nitrogen atom to which are attached both R'$_3$ and R'$_4$ forms part of a heterocyclic structure defined by R'$_4$-N-R'$_3$, on condition that at least 50% of the R'$_4$ represent a hydrogen atom, and
L represents a bonding group preferably chosen from ester, ether, amine, urea, urethane, thioester, thioether, thiourea and thiourethane, optionally substituted with at least one group R'$_1$ as defined above.

According to one embodiment, these polymers are chosen from the polymers of formula (A) in which the bonding group L represents an ester group

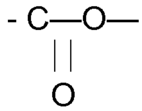

These polymers are more especially the ones described in document US-A-5 783 657 from the company Union Camp.
Each of these polymers in particular satisfies formula (B) below:

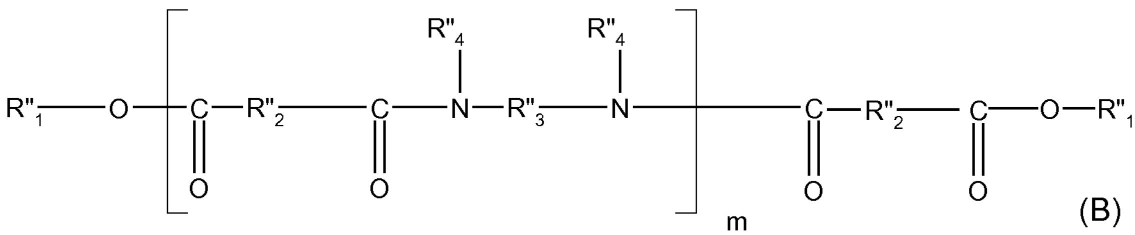

(B)

in which:

- m denotes a whole number of amide units such that the number of ester groups represents from 10% to 50% of the total number of ester and amide groups;
- R"$_1$ is independently in each case an alkyl or alkenyl group containing at least 4 carbon atoms and especially from 4 to 24 carbon atoms;
- R"$_2$ represents independently in each case a $C_4$ to $C_{42}$ hydrocarbon-based group, on condition that 50% of the groups $R_2$ represent a $C_{30}$ to $C_{42}$ hydrocarbon-based group;
- R"$_3$ represents independently in each case an organic group bearing at least two carbon atoms, hydrogen atoms and optionally one or more oxygen or nitrogen atoms;
- and R"$_4$ represents independently in each case a hydrogen atom, a $C_1$ to $C_{10}$ alkyl group or a direct bond to R"$_3$ or to another $R_4$ such that the nitrogen atom to which are attached both R"$_3$ and R"$_4$ forms part of a heterocyclic structure defined by R"$_4$-N-R"$_3$, with at least 50% of the groups $R_4$ representing a hydrogen atom.

In the particular case of formula (B), the terminal fatty chains that are optionally functionalized for the purposes of the invention are terminal chains linked to the last nitrogen atom of the polyamide backbone.
In particular, the ester groups of formula (B), which form part of the terminal and/or pendent fatty chains within the meaning of the invention, represent from 15% to 40% and better still from 20% to 35% of the total number of ester and amide groups.
Furthermore, m advantageously represents an integer ranging from 1 to 5 and better still greater than 2.
Preferably, R"$_1$ is a $C_{12}$ to $C_{22}$ and preferably $C_{16}$ to $C_{22}$ alkyl group. Advantageously, R"$_2$ may be a $C_{10}$ to $C_{42}$ hydrocarbon-based (alkylene) group. Preferably, at least 50% and better still at least 75% of the groups R"$_2$ are groups containing from 30 to 42 carbon atoms. The other groups R"$_2$ are $C_4$ to $C_{19}$ and better still $C_4$ to $C_{12}$ hydrogen-containing groups.
Preferably, R"$_3$ represents a $C_2$ to $C_{36}$ hydrocarbon-based group or a polyoxyalkylene group and $R_4$ represents a hydrogen atom. Preferably, $R_3$ represents a $C_2$ to $C_{12}$ hydrocarbon-based group.
The hydrocarbon-based groups may be linear, cyclic or branched, and saturated or unsaturated groups. Moreover, the alkyl and alkylene groups may be linear or branched, and saturated or unsaturated groups.
In general, the polymers of formula (B) are in the form of mixtures of polymers, these mixtures also possibly

containing a synthetic product corresponding to a compound of formula (B) in which n is 0, i.e. a diester.

According to a particularly preferred form of the invention, use will be made of a mixture of copolymers of a $C_{36}$ diacid condensed onto ethylenediamine; the terminal ester groups result from the esterification of the remaining acid end groups with cetyl alcohol, stearyl alcohol or mixtures thereof (also known as cetylstearyl alcohol) (INCI name: Ethylenediamine/stearyl dimer dilinoleate copolymer). Its weight-average molecular mass is preferably 6000. These mixtures are especially sold by the company Arizona Chemical under the trade names Uniclear 80 and Uniclear 100 VG in the form, respectively, of a gel at 80% (of active material) in a mineral oil, and at 100% (of active material). They have a softening point of 88°C to 94°C.

As polyamide polycondensates corresponding to the general formula (A), mention may also be made of polymers comprising at least one terminal fatty chain bonded to the polymer backbone via at least one tertiary amide bonding group (also known as an amide-terminated polyamide or ATPA). For further information regarding these polymers, reference may be made to US 6 503 522.

According to a particularly preferred form of the invention, use will be made more particularly of a copolymer of hydrogenated linoleic diacid, of ethylenediamine and of di($C_{14}$-$C_{18}$)alkylamine(s) (INCI name: ETHYLENEDIAMIDE/HYDROGENATED DIMER DILINOLEATE COPOLYMER BIS-DI-$C_{14}$-$C_{18}$ ALKYL AMIDE). This copolymer is especially sold under the trade name Sylvaclear A200V by the company Arizona Chemical.

According to another embodiment, the polyamide of formula (A) may also be an ester-terminated poly(esteramide) (ETPEA), for instance those whose preparation is described in US 6 552 160.

According to one particularly preferred form of the invention, use will be made more particularly of a copolymer of hydrogenated linoleic diacid, of ethylenediamine and of neopentyl glycol and stearyl alcohol (INCI name: BIS-STEARYL ETHYLENEDIAMINE/NEOPENTYL GLYCOL/STEARYL HYDROGENATED DIMER DILINOLEATE COPOLYMER). This copolymer is especially sold under the trade name Sylvaclear C75 V by the company Arizona Chemical.

As polyamide polycondensates that may be used in the invention, mention may also be made of those comprising at least one terminal fatty chain bonded to the polymer backbone via at least one ether or polyether bonding group (it is then referred to as an ether-terminated poly(ether)amide). Such polymers are described, for example, in US 6 399 713.

The polyamides in accordance with the invention advantageously have a softening point of greater than 65°C, which may be up to 190°C. It preferably has a softening point ranging from 70°C to 130°C and better still from 80°C to 105°C. The polyamide is in particular a non-waxy polymer.

As polyamide polycondensates that may be used in the invention, mention may also be made of polyamide resins resulting from the condensation of an aliphatic dicarboxylic acid and a diamine (including compounds containing more than 2 carbonyl groups and 2 amine groups), the carbonyl and amine groups of adjacent individual units being condensed via an amide bond. These polyamide resins are especially the products sold under the brand name Versamid® by the companies General Mills, Inc. and Henkel Corp. (Versamid 930, 744 or 1655) or by the company Olin Mathieson Chemical Corp., under the brand name Onamid® especially Onamid S or C. These resins have a weight-average molecular mass ranging from 6000 to 9000. For further information regarding these polyamides, reference may be made to US 3 645 705 and US 3 148 125. Use is made more especially of Versamid® 930 or 744.

It is also possible to use the polyamides sold by the company Arizona Chemical under the references Uni-Rez (2658, 2931, 2970, 2621, 2613, 2624, 2665, 1554, 2623 and 2662) and the product sold under the reference Macromelt 6212 by the company Henkel. For further information regarding these polyamides, reference may be made to US 5 500 209.

It is also possible to use vegetable-based polyamide resins, for instance those described in patents US 5 783 657 and US 5 998 570.

## LINEAR $C_1$-$C_4$ MONOALCOHOL

The term "$C_1$-$C_4$ monoalkanol" means any linear or branched saturated alkane compound containing from 1 to 4 carbon atoms and only one hydroxyl (OH) function.

The $C_1$-$C_4$ monoalcohol(s) present in the compositions of the invention may be chosen from methanol, ethanol and propanol, or mixtures thereof. Ethanol will be chosen more particularly.

They are generally present in concentrations ranging from 0.1% to 40% by weight and more preferentially from 2% to 10% by weight relative to the total weight of the composition.

Another subject of the present invention consists of the use of the compositions according to the invention as defined above for the manufacture of products for the cosmetic treatment of the skin, the lips, the nails, the hair, the eyelashes, the eyebrows and/or the scalp, especially care products, antisun products and makeup products.

The cosmetic compositions according to the invention may be used, for example, as makeup products.

Another subject of the present invention consists of a non-therapeutic cosmetic process for caring for and/or making up a keratin material, which consists in applying, to the surface of the said keratin material, at least one composition according to the invention as defined above.

The cosmetic compositions according to the invention may be used, for example, as care products and/or antisun protection products for the face and/or the body, of liquid to solid consistency, such as lotions, more or less smooth gels, and pastes. They may optionally be packaged in aerosol form and may be in the form of a mousse or a spray.
The compositions according to the invention in the form of vaporizable fluid lotions in accordance with the invention are applied to the skin or the hair in the form of fine particles by means of pressurization devices. The devices in accordance with the invention are well known to those skilled in the art and comprise non-aerosol pumps or "atomizers", aerosol containers comprising a propellant and aerosol pumps using compressed air as propellant. These devices are described in patents US 4 077 441 and US 4 850 517.
The compositions packaged as an aerosol in accordance with the invention generally comprise conventional propellants, such as, for example, hydrofluoro compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

## ASSEMBLY

According to another aspect, the invention also relates to a cosmetic assembly comprising:

i) a container delimiting one or more compartment(s), the said container being closed by a closing member and optionally not being leaktight; and
ii) a makeup and/or care composition in accordance with the invention placed inside the said compartment(s).

The container may be, for example, in the form of a jar or a box.
The closing member may be in the form of a lid comprising a cap mounted so as to be able to move by translation or by pivoting relative to the container housing the said makeup and/or care composition(s).
The examples that follow serve to illustrate the invention without, however, being limiting in nature. In these examples, the amounts of the composition ingredients are given as % by weight, relative to the total weight of the composition.

## Example A1: Preparation of compound (14)

(14)

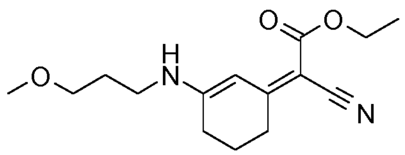

122.23 g of 3-[(3-methoxypropyl)amino]-2-cyclohexen-1-one were alkylated with dimethyl sulfate or alternatively with diethyl sulfate and treated with 75.45 g of ethyl cyanoacetate in approximately equimolar proportions in the presence of a base and optionally of a solvent.
The following base/solvent combinations were used:

| Example | Base | Solvent |
|---|---|---|
| Example A1.1 | DBU (1,8-diazabicyclo[5.4.0]undec-7-ene) | dimethylacetamide |
| Example A1.2 | triethylamine | isopropanol |
| Example A1.3 | 3-methoxypropylamine | isopropanol |
| Example A1.4 | 3-methoxypropylamine | tert-amyl alcohol |
| Example A1.5 | 3-methoxypropylamine | toluene |
| Example A1.6 | 3-methoxypropylamine | dimethylformamide |
| Example A1.7 | 3-methoxypropylamine | no solvent |
| Example A1.8 | N-morpholine | isopropanol |

The completion of the alkylation reaction was monitored, for example, via methods such as TLC, GC or HPLC.
162.30 g of compound (14) were obtained in the form of a brown oil.
After crystallization, the product was obtained in the form of yellowish crystals. Melting point: 92.7°C.

**Example A2: Preparation of compound (15)**

(15) 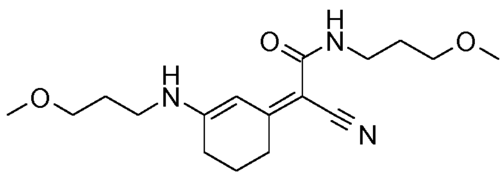

101.00 g of 3-[(3-methoxypropyl)amino]-2-cyclohexen-1-one were alkylated with dimethyl sulfate or alternatively with diethyl sulfate and treated with 86.00 g of 2-cyano-N-(3-methoxypropyl)acetamide in approximately equimolar proportions in the presence of a base and optionally of a solvent.
The following base/solvent combinations were used:

| Example | Base | Solvent |
|---|---|---|
| Example A2.1 | DBU (1,8-diazabicyclo[5.4.0]undec-7-ene) | dimethylacetamide |
| Example A2.2 | triethylamine | isopropanol |
| Example A2.3 | 3-methoxypropylamine | isopropanol |
| Example A2.4 | 3-methoxypropylamine | tert-amyl alcohol |
| Example A2.5 | 3-methoxypropylamine | toluene |
| Example A2.6 | 3-methoxypropylamine | dimethylformamide |
| Example A2.7 | 3-methoxypropylamine | no solvent |

The crude product (15) is obtained in the form of a dark brown oil.
After chromatography on a column of silica gel (eluent: 99/1 toluene/methanol), 81.8 g of product are obtained in the form of yellowish crystals. Melting point: 84.7-85.3°C.

**Example A3: Preparation of compound (27)**

(27) 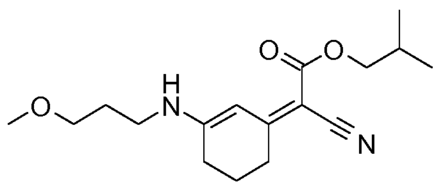

13.09 g of 3-[(3-methoxypropyl)amino]-2-cyclohexen-1-one are alkylated with dimethyl sulfate or alternatively with diethyl sulfate and treated with 10.12 g of isobutyl cyanoacetate in approximately equimolar proportions in the presence of a base and optionally of a solvent.
The following base/solvent combinations are used:

| Example | Base | Solvent |
|---|---|---|
| Example A3.1 | DBU (1,8-diazabicyclo[5.4.0]undec-7-ene) | dimethylacetamide |
| Example A3.2 | triethylamine | isopropanol |

(continued)

| Example | Base | Solvent |
|---|---|---|
| Example A3.3 | 3-methoxypropylamine | isopropanol |
| Example A3.4 | N-methylmorpholine | tert-amyl alcohol |
| Example A3.5 | 3-methoxypropylamine | toluene |
| Example A3.6 | 3-methoxypropylamine | dimethylformamide |
| Example A3.7 | 3-methoxypropylamine | no solvent |

15.97 g of the crude product (27) are obtained in the form of a dark brown oil.

After chromatography on a column of silica gel (eluent: toluene/acetone), 13.46 g of product are obtained in the form of yellowish crystals.
Melting point: 96.3°C.

**Example A4: Preparation of compound (25)**

(25)

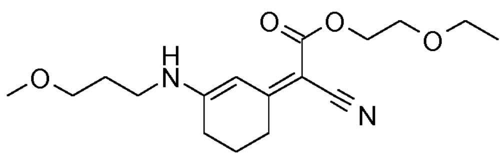

148.4 g of 3-[(3-methoxypropyl)amino]-2-cyclohexen-1-one were alkylated with dimethyl sulfate or alternatively with diethyl sulfate and treated with 130.00 g of 2-ethoxyethyl cyanoacetate in the presence of a base and a solvent.

The following base/solvent combinations were used:

| Example | Base | Solvent |
|---|---|---|
| Example A4.1 | DBU (1,8-diazabicyclo[5.4.0]undec-7-ene) | dimethylacetamide |
| Example A4.2 | triethylamine | isopropanol |
| Example A4.3 | 3-methoxypropylamine | isopropanol |
| Example A4.4 | N-methylmorpholine | tert-amyl alcohol |
| Example A4.5 | 3-methoxypropylamine | toluene |
| Example A4.6 | 3-methoxypropylamine | dimethylformamide |
| Example A4.7 | 3-methoxypropylamine | no solvent |

**Formulation Examples 1 to 4**

The formulations 1 to 4 below were prepared, in which the chemical stability of compound (25) of the invention was evaluated. The amounts are expressed in % weight relative to the total weight of the composition. The formulation 3 was constructed such that the proportion by weight of each ingredient was identical to the proportion by weight of each ingredient in the fatty phase of the formulation 4.

| Phase | Ingredients | Formulation 1 (invention) Oil | Formulation 2 (outside the invention) Emulsion |
|---|---|---|---|
| A | $C_{12-15}$ ALKYL BENZOATE | qs 100 | - |
| | ISOPROPYL PALMITATE | 25 | - |
| | LIQUID PARAFFIN | 14 | - |
| | OCTYLDODECANOL | 14 | - |
| | ETHYLENEDIAMINE/STEARYL DIMER DILINOLEATE COPOLYMER (UNICLEAR 100 VG® - ARIZONA CHEMICAL) | 6 | - |
| | Compound (25) | 0.5 | 0.5 |
| | | | |
| | PHENETHYL BENZOATE (and) BENZOIC ACID (X-TEND 226® - ISP) | - | 30 |
| | STEARIC ACID | - | 1.5 |
| | GLYCERYL STEARATE (and) PEG-100 STEARATE (ARLACEL 165®) | - | 2.5 |
| | DIMETHICONE | - | 0.5 |
| | CETYL ALCOHOL | - | 0.5 |
| | CETEARYL ALCOHOL (and) CETEARYL GLUCOSIDE (MONTANOV 68® - SEPPIC) | - | 2 |
| | PRESERVATIVE | - | 1 |
| B | WATER | - | qs 100 |
| | GLYCERIN | - | 5 |
| | TRIETHANOLAMINE | - | 0.45 |
| | DISODIUM EDTA | - | 0.1 |
| | POTASSIUM CETYL PHOSPHATE (AMPHISOL K® -DSM NUTRITIONAL PRODUCTS) | - | 1 |
| C | ISOHEXADECANE | - | 1 |
| | ACRYLATES/C10-30 ALKYL ACRYLATE CROSSPOLYMER (PEMULEN TR1) | - | 0.2 |
| | XANTHAN GUM | - | 0.2 |
| D | ETHANOL | 8 | - |
| | WATER | - | 1.35 |
| | TRIETHANOLAMINE | - | 0.2 |

| Phase | Ingredients | Formulation 3 (invention) Oil | Formulation 4 (outside the invention) Emulsion |
|---|---|---|---|
| A | Isopropyl Lauroyl Sarcosinate (Eldew SL-205®) | 73.17 | 30 |
| | Compound (25) | 4.88 | 2 |
| | Stearic Acid | 3.66 | 1.5 |
| | Glyceryl Stearate (and) PEG-100 Stearate (Arlacel 165®) | 6.10 | 2.5 |
| | Cetyl Alcohol | 1.22 | 0.5 |
| | Cetearyl Alcohol (and) Cetearyl Glucoside (Montanov 68®) | 4.88 | 2 |
| | Dimethicone | 1.22 | 0,5 |
| | Preservative | 2.44 | 1 |
| B | Water | - | qsp 100 |
| | Glycerin | - | 5 |
| | Triethanolamine | - | 0.45 |
| | Disodium EDTA | - | 0.1 |
| | Potassium Cetyl Phosphate (Amphisol K®) | - | 1 |
| C | Isohexadecane | 2.44 | 1 |
| | Acrylates / C10-30 Alkyl Acrylate crosspolymer (Pemulen TR1) | - | 0.2 |
| | Xanthan gum | - | 0.2 |
| D | Triethanolamine | - | 0.2 |
| **% compound (25) after 1 month at 45° C** | | **4.85** | **1.78** |
| **% Loss of compound (25) after 1 month at 45° C relative to the theoritical content** | | **0.6** | **11** |

**Method for preparing formulationd 1 and 3 (oil):**

The oily formulations of the invention were prepared in the following manner. Phase A was heated to 80°C with stirring. Once this phase was clear, the alcohol (phase D) was added at room temperature with continued stirring using a magnetic stirrer for between 10 minutes and 1 hour.

**Method for preparing formulations 2 and 4 (emulsion):**

The oily phase A and aqueous phase B were prepared by mixing the starting materials with mechanical stirring at 80°C. Once the oily solution A and aqueous solution B were macroscopically homogeneous, the emulsion outside the invention was prepared by introducing phase A into phase B, both of which were stirred using a rotor-stator homogenizer (mixer in the case of the invention). Stirring was maintained for 10 to 20 minutes before adding phase C, with continued stirring. The emulsion was cooled to room temperature before adding the ingredients of phase D.

**Protocol for evaluating the merocyanine stability:**

The stability of the merocyanines in formulation was evaluated by UPLC assay of the residual merocyanine content after 2 months of storage of the formulations at 4° and 45°C.
The percentage of degradation of the merocyanines after 2 months at 45°C is expressed as:

$$\text{Degradation}_{t2M45°C}(\%) = \frac{\text{Merocyanine}_{t2M45°C} - \text{Merocyanine}_{t2M4°C}}{\text{Merocyanine}_{t2M4°C}} \times 100$$

The percentage of degradation of the merocyanines after 1 month at 45°C is expressed as:

$$\text{Degradation}_{t1M45°C}(\%) = \frac{\text{Merocyanine}_{t1M45°C} - \text{Merocyanine}_{theoritical}}{\text{Merocyanine}_{theoritical}} \times 100$$

The theoritical content corresponds to the initial introduced content.

| **Evaluated chemical stability** | **Formulation 1 (invention)** | **Formulation 2 (outside the invention)** |
|---|---|---|
| Residual merocyanine after 2 months at 4°C (%) | 0.54 | 0.49 |
| Residual merocyanine after 2 months at 45°C (%) | 0.54 | 0.46 |
| Merocyanine degradation after 2 months at 45°C (%) | < 1% | 8% |
| | | |
| **Evaluated chemical stability** | **Formulation 3 (invention)** | **Formule 4 (outside the invention)** |
| Theoritical content (%) | 4.88 | 2 |
| Residual merocyanine after 1 month at 45°C (%) | 4.85 | 1.78 |
| Merocyanine degradation after 1 month at 45°C (%) | 0.6 | 11 |

The stability results obtained on formulations 1 and 3 according to the invention in comparison with formulations 2 and 4 show that the merocyanines of the invention, formulated in anhydrous compositions of the invention, are chemically more stable than when they are formulated in a support comprising an emulsifying system containing at least one alkali metal salt of a phosphoric acid ester of a fatty alcohol.

## Claims

**1.** Anhydrous cosmetic or dermatological composition comprising, in a physiologically acceptable support:

a) at least one merocyanine compound corresponding to the following formula (3) and also the E/E- or E/Z-geometrical isomer forms thereof:

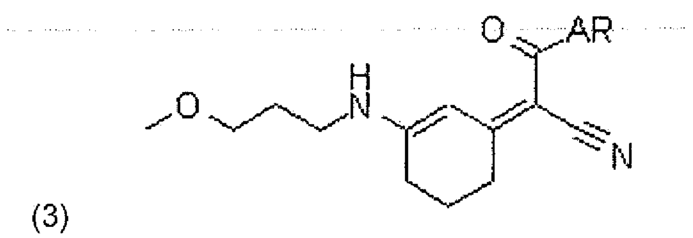

(3)

in which:

A is -O- or -NH;
R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, it being possible for the said groups to be interrupted with one or more O, and

b) at least one oily phase comprising at least one oil chosen from hydrocarbon-based oils, silicone oils and fluoro oils, and mixtures thereof.

**2.** Composition according to Claim 1, in which the merocyanines of formula (3) are chosen from the following compounds and also the E/E- or E/Z- geometrical isomer forms thereof:

| | |
|---|---|
| 14 | 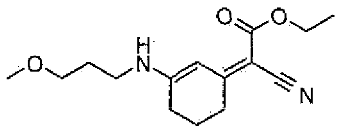<br>ethyl (2Z)-cyano{3-[(3-methoxypropyl)amino]cyclohex-2-en-1-ylidene}ethanoate |
| 15 | 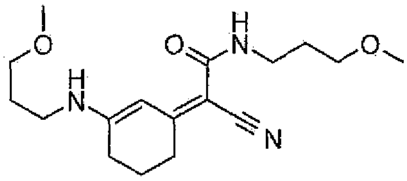<br>(2Z)-2-cyano-N-(3-methoxypropyl)-2-{3-[(3-methoxypropyl)amino]cyclohex-2-en-1-ylidene}ethanamide |
| 25 | 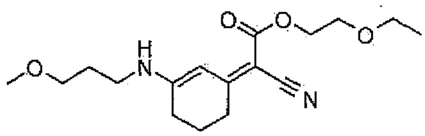<br>2-ethoxyethyl (2Z)-cyano{3-[(3-methoxypropyl)amino]cyclohex-2-en-1-ylidene}ethanoate |
| 27 | 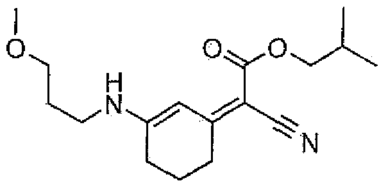<br>2-methylpropyl (2Z)-cyano{3-[(3-methoxypropyl)amino]cyclohex-2-en-1-ylidene}ethanoate |
| 29 | 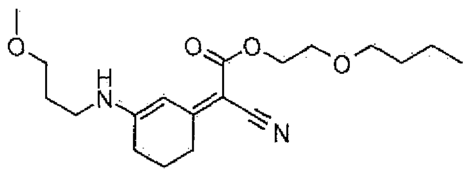<br>2-butoxyethyl (2Z)-cyano{3-[(3-methoxypropyl)amino]cyclohex-2-en-1-ylidene}ethanoate |
| 31 | 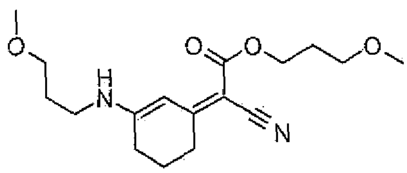<br>3-methoxypropyl (2Z)-cyano{3-[(3-methoxypropyl)amino]cyclohex-2-en-1-ylidene}ethanoate |
| 37 | 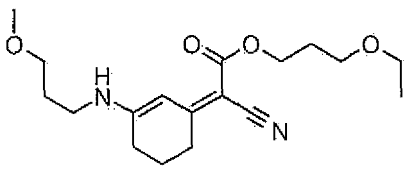 |
| | 3-ethoxypropyl (2Z)-cyano{3-[(3-methoxypropyl)amino]cyclohex-2-en-1-ylidene}ethanoate |

3. Composition according to Claim 2, in which the merocyanine of formula (3) is the compound 2-ethoxyethyl (2Z)-cyano{3-[(3-methoxypropyl)amino]cyclohex-2-en-1-ylidene}ethanoate (25) in its E/Z geometrical configuration having the following structure:

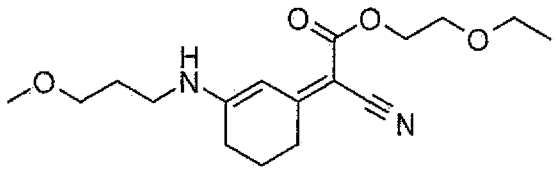

and/or the E/E form having the following structure:

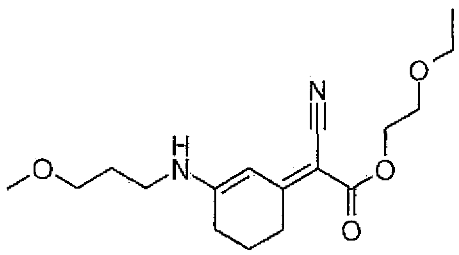

4. Composition according to any one of Claims 1 to 3, also comprising at least one water-insoluble UV-screening agent.

5. Composition according to any one of the preceding claims, in which the overall oily phase represents from 2% to 100% by weight and preferentially from 10% to 90% by weight relative to the total weight of the composition.

6. Composition according to any one of the preceding claims, **characterized in that** it is transparent and has a turbidity at 25°C of less than 1000 NTU, preferably less than 50 NTU and even more preferentially less than 15 NTU.

7. Composition according to any one of the preceding claims, **characterized in that** it is in the form of fluid composition and also comprises:

a) at least one linear $C_1$-$C_4$ monoalkanol and
b) at least one lipophilic polyamide polycondensate.

8. Composition as defined in any one of claims 1 to 7 for use in a process for caring for and/or making up a keratin material, comprising the application, to the surface of the said keratin material.

9. Composition as defined in any one of claims 1 to 7 for use in a non-therapeutic cosmetic process for limiting the darkening of the skin and/or improving the colour and/or uniformity of the complexion, comprising the application, to the surface of the skin.

10. Composition as defined in any one of claims 1 to 7 for use in a process for preventing and/or treating the signs of ageing of a keratin material, comprising the application, to the surface of the keratin material.

## Patentansprüche

1. Wasserfreie kosmetische oder dermatologische Zusammensetzung, umfassend in einem physiologisch unbedenklichen Träger:

a) mindestens eine Merocyaninverbindung, die der folgenden Formel (3) entspricht, sowie die geometrischen E/E- oder E/Z-Isomerformen davon:

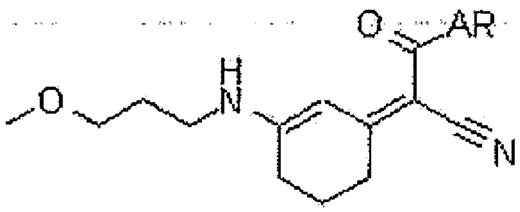

(3)

wobei:

A für -O- oder -NH steht;
R für eine $C_1$-$C_{22}$-Alkylgruppe, eine $C_2$-$C_{22}$-Alkenylgruppe, eine $C_2$-$C_{22}$-Alkinylgruppe, eine $C_3$-$C_{22}$-Cycloalkylgruppe oder eine $C_3$-$C_{22}$-Cycloalkenyl-gruppe, wobei diese Gruppen durch ein oder mehrere O unterbrochen sein können, steht, und

b) mindestens eine ölige Phase, die mindestens ein Öl, das aus Ölen auf Kohlenwasserstoffbasis, Silikonölen und Fluorölen und Mischungen davon ausgewählt ist, umfasst.

**2.** Zusammensetzung nach Anspruch 1, wobei die Merocyanine der Formel (3) aus den folgenden Verbindungen sowie den geometrischen E/E- oder E/Z-Isomerformen davon ausgewählt sind:

| | |
|---|---|
| 14 | 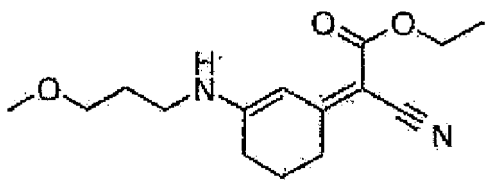<br>(2Z)-Cyano{3-[(3-methoxypropyl)amino]-cyclohex-2-en-1-yliden}ethansäureethylester |
| 15 | 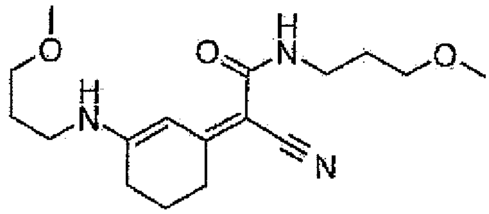<br>(2Z)-2-Cyano-N-(3-methoxypropyl)-2-{3-[(3-methoxypropyl)amino]cyclohex-2-en-1-yliden}ethanamid |
| 25 | 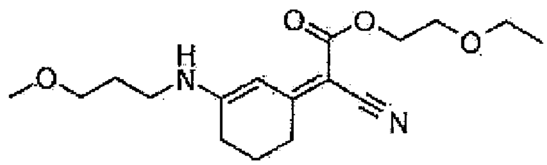<br>(2Z)-Cyano{3-[(3-methoxypropyl)amino]-cyclohex-2-en-1-yliden}ethansäure-2-ethoxyethylester |
| 27 | 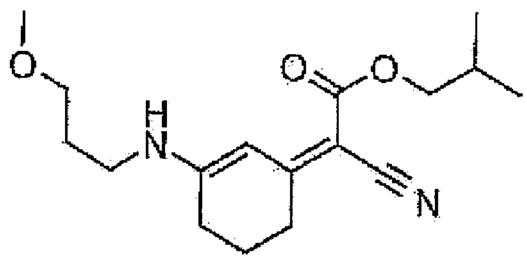<br>(2Z)-Cyano{3-[(3-methoxypropyl)amino]-cyclohex-2-en-1-yliden}ethansäure-2-methylpropylester |

(fortgesetzt)

| | |
|---|---|
| 29 | 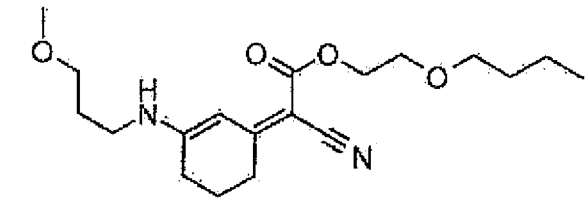<br>(2Z)-Cyano{3-[(3-methoxypropyl)amino]-cyclohex-2-en-1-yliden}ethansäure-2-butoxyethylester |
| 31 | 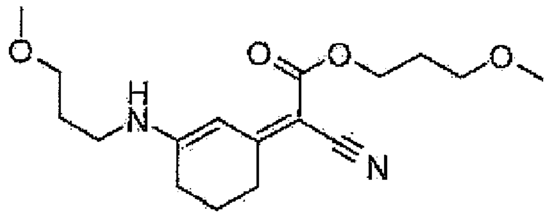 |
| | (2Z)-Cyano{3-[(3-methoxypropyl)amino]-cyclohex-2-en-1-yliden}ethansäure-3-methoxypropylester |
| 37 | 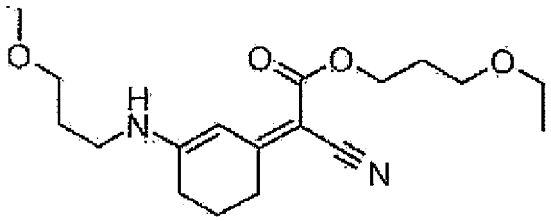<br>(2Z)-Cyano{3-[(3-methoxypropyl)amino]-cyclohex-2-en-1-yliden}ethansäure-3-ethoxypropylester |

**3.** Zusammensetzung nach Anspruch 2, wobei es sich bei dem Merocyanin der Formel (3) um die Verbindung (2Z)-Cyano{3-[(3-methoxypropyl)amino]cyclohex-2-en-1-yliden}ethansäure-2-ethoxyethylester (25) in ihrer geometrischen E/Z-Konfiguration mit der folgenden Struktur:

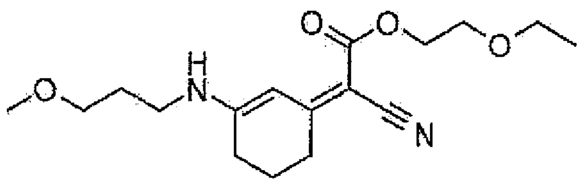

und/oder die E/E-Form mit der folgenden Struktur:

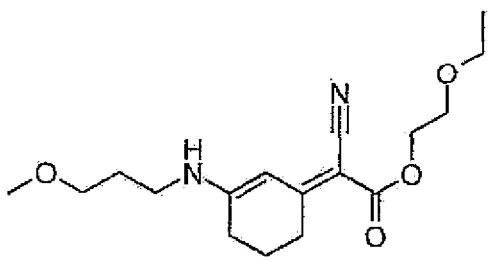

handelt.

**4.** Zusammensetzung nach einem der Ansprüche 1 bis 3, die außerdem mindestens eine wasserunlösliche UV-Filtersubstanz umfasst.

**5.** Zusammensetzung nach einem der vorhergehenden Ansprüche, wobei die gesamte ölige Phase 2 bis 100 Gew.-

% und vorzugsweise 10 bis 90 Gew.-%, bezogen auf das Gesamtgewicht der Zusammensetzung, ausmacht.

**6.** Zusammensetzung nach einem der vorhergehenden Ansprüche, **dadurch gekennzeichnet, dass** sie transparent ist und eine Trübung bei 25 °C von weniger als 1000 NTU, bevorzugt weniger als 50 NTU und noch weiter bevorzugt weniger als 15 NTU aufweist.

**7.** Zusammensetzung nach einem der vorhergehenden Ansprüche, **dadurch gekennzeichnet, dass** sie in Form einer fluiden zusammensetzung vorliegt und außerdem

a) mindestens ein lineares $C_1$-$C_4$-Monoalkanol und
b) mindestens ein lipophiles Polyamidpolykondensat umfasst.

**8.** Zusammensetzung gemäß einem der Ansprüche 1 bis 7 zur Verwendung bei einem Verfahren zum Pflegen und/oder Schminken eines Keratinmaterials, umfassend das Aufbringen auf die Oberfläche des Keratinmaterials.

**9.** Zusammensetzung gemäß einem der Ansprüche 1 bis 7 zur Verwendung bei einem nichttherapeutischen kosmetischen Verfahren zum Einschränken des Dunkelwerdens der Haut und/oder zum Verbessern der Farbe und/oder Einheitlichkeit des Teints, umfassend das Aufbringen auf die Oberfläche der Haut.

**10.** Zusammensetzung gemäß einem der Ansprüche 1 bis 7 zur Verwendung bei einem Verfahren zum Verhindern und/oder Behandeln der Anzeichen von Alterung eines Keratinmaterials, umfassend das Aufbringen auf die Oberfläche des Keratinmaterials.

## Revendications

**1.** Composition cosmétique ou dermatologique anhydre comprenant, dans un support physiologiquement acceptable :

a) au moins un composé de mérocyanine correspondant à la formule (3) suivante et en outre les formes d'isomère géométrique E/E ou E/Z de celui-ci :

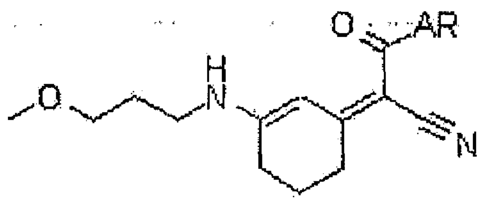

(3)

dans lequel :

A est -O- ou -NH ;
R est un groupe alkyle en $C_1$-$C_{22}$, un groupe alcényle en $C_2$-$C_{22}$, un groupe alcynyle en $C_2$-$C_{22}$, un groupe cycloalkyle en $C_3$-$C_{22}$ ou un groupe cycloalcényle en $C_3$-$C_{22}$, lesdits groupes pouvant être interrompus par un ou plusieurs O, et

b) au moins une phase huileuse comprenant au moins une huile choisie parmi des huiles à base d'hydrocarbure, des huiles de silicone et des huilés fluorées, et des mélanges de celles-ci.

**2.** Composition selon la revendication 1, dans laquelle les mérocyanines de formule (3) sont choisies parmi les composés suivants ainsi que les formes d'isomère géométrique E/E ou E/Z de ceux-ci :

| | |
|---|---|
| 14 | 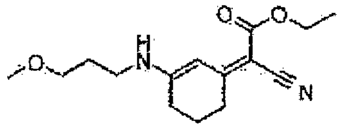<br>(2Z)-cyano{3-[(3-méthoxypropyl)amino]-cyclohex-2-én-1-ylidène}-éthanoate d'éthyle |
| 15 | 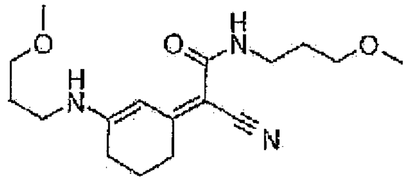<br>(2Z)-2-cyano-N-(3-méthoxypropyl)-2-{3-[(3-méthoxypropyl)amino]-cyclohex-2-én-1-ylidène}-éthanamide |
| 25 | 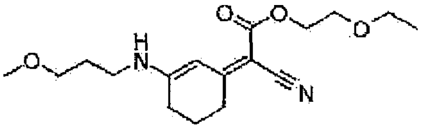<br>(2Z)-cyano{3-[(3-méthoxypropyl)amino]-cyclohex-2-én-1-ylidène}-éthanoate de 2-éthoxyéthyle |
| 27 | 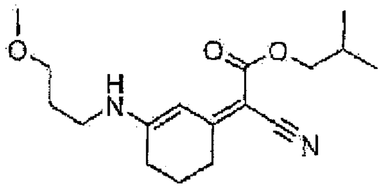<br>(2Z)-cyano{3-[(3-méthoxypropyl)amino]-cyclohex-2-én-1-ylidène}-éthanoate de 2-méthylpropyle |
| 29 | 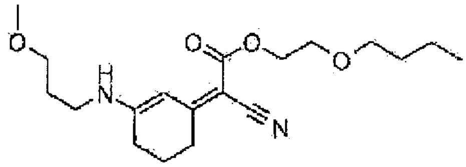 |
| | (2Z)-cyano{3-[(3-méthoxypropyl)amino]-cyclohex-2-én-1-ylidène}-éthanoate de 2-butoxyéthyle |
| 31 | 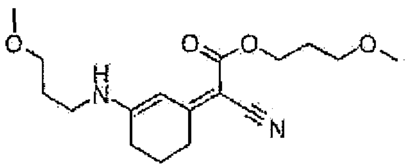<br>(2Z)-cyano{3-[(3-méthoxypropyl)amino]-cyclohex-2-én-1-ylidène}-éthanoate de 3-méthoxypropyle |
| 37 | 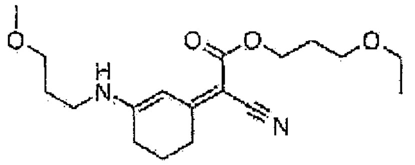<br>(2Z)-cyano{3-[(3-méthoxypropyl)amino]-cyclohex-2-én-1-ylidène}-éthanoate dé 3-éthoxypropyle |

**3.** Composition selon la revendication 2, dans laquelle la mérocyanine de formule (3) est le composé (2Z)-cyano{3-[(3-méthoxypropyl)amino]cyclohex-2-én-1-ylidène}éthanoate de 2-éthoxyéthyle (25) dans sa configuration géométrique E/Z ayant la structure suivante :

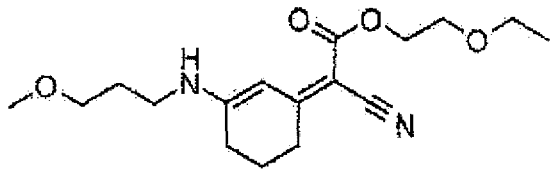

et/ou la forme E/E ayant la structure suivante :

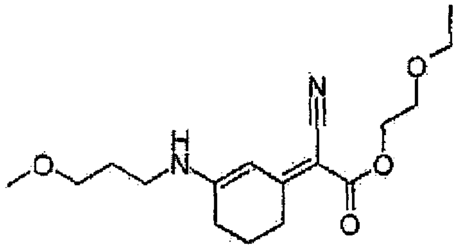

**4.** Composition selon l'une quelconque des revendications 1 à 3, comprenant en outre au moins un agent anti-UV insoluble dans l'eau.

**5.** Composition selon l'une quelconque des revendications précédentes, dans laquelle la phase huileuse totale représente de 2 % à 100 % en poids et de préférence, de 10 % à 90 % en poids par rapport au poids total de la composition.

**6.** Composition selon l'une quelconque des revendications précédentes, **caractérisée en ce qu'**elle est transparente et présente une turbidité à 25 °C inférieure à 1000 NTU, de préférence inférieure à 50 NTU et, encore plus préférablement, inférieure à 15 NTU.

**7.** Composition selon l'une quelconque des revendications précédentes, **caractérisée en ce qu'**elle est sous la forme d'une composition fluide et comprend en outre :

a) au moins un monoalcanol en $C_1$-$C_4$ linéaire et
b) au moins un polycondensat de polyamide lipophile.

**8.** Composition telle que définie dans l'une quelconque des revendications 1 à 7 pour utilisation dans un procédé de soin et/ou de maquillage d'un matériau kératinique, comprenant l'application, sur la surface dudit matériau kératinique.

**9.** Composition telle que définie dans l'une quelconque des revendications 1 à 7 pour utilisation dans un procédé cosmétique non thérapeutique pour limiter l'assombrissement de la peau et/ou améliorer la couleur et/ou l'uniformité de la complexion, comprenant l'application, sur la surface de la peau.

**10.** Composition telle que définie dans l'une quelconque des revendications 1 à 7 pour utilisation dans un procédé de prévention et/ou traitement des signes de vieillissement d'un matériau de kératine, comprenant l'application, sur la surface du matériau kératinique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4195999 A
- WO 2004006878 A
- WO 2008090066 A
- WO 2011113718 A
- WO 2009027258 A
- WO 2013010590 A
- WO 2013011094 A
- WO 2013011480 A
- US 20030181483 A1
- WO 0234710 A
- WO 2007071582 A
- US 4749643 A
- WO 2007068371 A
- WO 2008155059 A
- US 5624663 A
- US 5237071 A
- US 5166355 A
- GB 2303549 A
- DE 19726184
- EP 893119 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 2007071584 A
- WO 2009063392 A
- US 6225467 B
- WO 2004085412 A
- WO 06035000 A
- WO 06034982 A
- WO 06034991 A
- WO 06035007 A
- WO 2006034992 A
- WO 2006034985 A
- EP 0841341 A
- EP 0518773 A
- US 5783657 A
- US 6503522 B
- US 6552160 B
- US 6399713 B
- US 3645705 A
- US 3148125 A
- US 5500209 A
- US 5998570 A
- US 4077441 A
- US 4850517 A


### Non-patent literature cited in the description


- *IP COM JOURNAL N°000179675D,* 23 February 2009
- *IP COM JOURNAL N°000182396D*
- *IP COM JOURNAL N° 000189542D,* 12 November 2009
- *IP COM Journal N°IPCOM000011179D,* 03 April 2004
- *J. Org. Chem. USSR (English translation),* 1990, vol. 26 (8), 1562f
- *J. Heterocycl. Chem.,* 1996, vol. 33 (3), 763-766
- *Khimiya Geterotsiklicheskikh Soedinenii,* 1984, vol. 11, 1537-1543
- *Khimiya Geterotsiklicheskikh Soedinenii,* 1982, vol. 3, 397-404
- *Chem.Heterocycl.Comp. (English translation),* 1988, vol. 24 (8), 914-919
- *Synthetic Communications,* 2003, vol. 33 (3), 367-371
- *Eur. J. Org. Chem.,* 2003, 2250-2253
- *J. Med. Chem.,* 1996, vol. 39, 1112-1124
- *J. Org. Chem.,* 1972, vol. 37 (8), 1141-1145
- *J. Heterocyclic Chem.,* 1990, vol. 27, 1143-1151
- *IP.com Journal,* 2009, vol. 9 (5A), 29-30
- *CHEMICAL ABSTRACTS,* 919803-06-8
- Symmetrical Triazine Derivatives. Symmetrical Triazine Derivatives. IP.COM IPCOM000031257 Journal, INC, 20 September 2004